# EUROPÄISCHE PATENTSCHRIFT

(11) **EP 3 291 855 B1**
(45) Veröffentlichungstag und Bekanntmachung des Hinweises auf die Patenterteilung: **29.06.2022**
(21) Anmeldenummer: 16724285.8
(22) Anmeldetag: 09.05.2016
(51) Int. Cl.: A61M 1/16, B01D 63/02, B01D 63/04

(54) **VORRICHTUNG MIT EINLASSABSCHNITT ZUR BEHANDLUNG EINER BIOLOGISCHEN FLÜSSIGKEIT**
DEVICE WITH INLET SECTION FOR TREATMENT OF A BIOLOGICAL LIQUID
DISPOSITIF DOTÉ DE SECTION D'ADMISSION DESTINÉ AU TRAITEMENT D'UN LIQUIDE BIOLOGIQUE

(30) Priorität: 07.05.2015 EP 15001369
(43) Veröffentlichungstag der Anmeldung: 14.03.2018
(73) Patentinhaber: Novalung GmbH, 74076 Heilbronn (DE)
(72) Erfinder: MATHEIS, Georg, 74076 Heilbronn (DE); METZGER, Fabian, 71296 Heimsheim (DE); BOGENSCHÜTZ, Josef, 72406 Bisingen (DE); ROßBROICH, Ralf, 52068 Aachen (DE)
(74) Vertreter: Graf von Stosch, Andreas
(86) Internationale Anmeldenummer: PCT/EP2016/000750
(87) Internationale Veröffentlichungsnummer: WO 2016/177476

(56) Entgegenhaltungen:
- EP-A1- 0 376 298
- WO-A1-2009/110652
- WO-A1-2009/155248
- WO-A1-2014/183852
- GB-A- 1 480 406
- US-A1- 2012 321 512

## Beschreibung

### Hintergrund der Erfindung

Die Erfindung betrifft eine Vorrichtung zur Behandlung einer biologischen Flüssigkeit gemäß dem Oberbegriff des unabhängigen Anspruchs und insbesondere eine Vorrichtung mit einer Kammer, die zur Aufnahme der biologischen Flüssigkeit bestimmt ist, und einem Gasaustauschmittel.

Dabei kann es sich um eine Begasungs- bzw. Entgasungsvorrichtung handeln, bei der ein oder mehrere Gase von einem Medium in ein anderes Medium übertreten können, oder eine Gasaustauschvorrichtung, die den Austausch eines oder mehrerer Gase zwischen zwei Medien ermöglicht. Solche Vorrichtungen finden Anwendung in der Chemie, der Biotechnologie und der Medizin. Ein wichtiger Einsatzzweck in der Medizin ist die Anreicherung einer biologischen Flüssigkeit, insbesondere von Blut, mit Sauerstoff und/oder die Entfernung von Kohlenstoffdioxid aus der Flüssigkeit, speziell Blut. Solche Maßnahmen sind bspw. bei der Behandlung von verschiedenen Lungenerkrankungen notwendig. Solche Maßnahmen können weiterhin z.B. auch bei akutem Lungenversagen, sowie zum Ersatz der Lunge während deren Umgehung mit einem extrakorporalen Kreislauf, mit unterschiedlichem Ausmaß bei der mechanischen Herzunterstützung und zur Ermöglichung am stillgestellten Herzen zu operieren, notwendig sein.

Zudem stellt die derzeit einzige langfristig effektive Therapieoption für Patienten mit endgradiger funktioneller Lungenerkrankung die Lungentransplantation dar. Eine andere medizinische Lösung, um dauerhaft die Funktion der Lunge zu ersetzen, existiert hingegen nicht. Bei Patienten, die unter chronischen Lungenerkrankungen leiden und nicht oder nicht unmittelbar für eine Lungentransplantation in Betracht kommen, besteht daher ein Bedürfnis nach künstlichen Lungenersatzverfahren.

Um ein derartiges Lungenersatzverfahren zu ermöglichen, sind so genannte Blutgastauscher aus dem Stand der Technik bekannt.

Ein Blutgastauscher, häufig auch als Oxygenator oder künstliche Lunge bezeichnet, wird entweder zur vollständigen, kurzzeitigen Übernahme der Lungenfunktion während einer Operation am offenen Herzen oder als vollständige oder teilweise, langfristige Unterstützung der Lunge auf der Intensivstation eingesetzt. Die Hauptfunktion eines solchen Blutgastauschers besteht in der Abgabe von Sauerstoff an das Blut (Oxygenierung) und in der Aufnahme von Kohlenstoffdioxid aus dem Blut (Decarboxylierung). Der Gasaustausch findet beispielsweise mittels Hohlfasermembranen statt, die in einem extrakorporalen Gasaustauscher von Blut umflossen werden, während gleichzeitig durch das Innere der Faser sauerstoffreiches bzw. kohlenstoffdioxidarmes Gas geleitet wird. Durch den Konzentrationsunterschied können Sauerstoff bzw. Kohlenstoffdioxid in jeweils entgegengesetzter Richtung durch eine semi-permeable Membran - typischerweise eine gaspermeable Membran - diffundieren. Die Fasern lassen sich kommerziell beziehen und werden beispielsweise in viereckigen Fasermatten oder als Einzelfaser auf Spulen gewickelt ausgeliefert.

Es gibt zwei verschiedene Herstellungsverfahren, um Blutgastauscher aus den Fasermatten herzustellen, nämlich gewickelt und gelegt. Beispielhaft soll hier nur eine Fasermattenherstellung anhand der gelegten Variante skizziert werden, um die Hintergründe der Erfindung besser zu verdeutlichen.

Bei der gelegten Variante werden die individuellen Fasermatten kreuzweise geschichtet, wodurch sich ein quaderförmiges, insbesondere ein kubisches, paketförmiges Faserbündel ergibt. Das Faserbündel wird durch zwei Deckel, welche die Anströmgeometrie enthalten, und durch eine Vergussmasse nach außen hin abgegrenzt. Die Deckel werden unterhalb und oberhalb des Faserbündels platziert. Die vier Seiten des Faserbündels werden auf einer Zentrifuge Seite für Seite nacheinander vergossen und so mit den Deckeln verbunden. Kommerziell erhältliche Gasaustauscher, die auf diese Weise hergestellt werden, besitzen weiterhin eine entsprechende kubische Kavität, in welche die Fasern eingebettet sind und vom Blut umströmt werden. Im Allgemeinen werden diese Gasaustauscher an einer der vier Ecken des Faserbündels bzw. der Kavität angeströmt. Die Anschlüsse für die blutführenden Schläuche sind dabei orthogonal zu den Deckeln der Gasaustauscher orientiert.

Auf Grund ihrer Größe und der Anordnung der Anschlüsse für die blutführenden Schläuche beschränkt sich der Einsatz dieser Gasaustauscher jedoch auf stationäre Anwendungen, bei denen der Patient - wach oder sediert - im Bett liegt. Da Patienten mit chronischen Lungenerkrankungen, die auf einen dieser bekannten Gasaustauscher angewiesen sind, somit stark in ihrer Mobilität eingeschränkt sind, ist nicht nur die Lebensqualität der Patienten erheblich vermindert. Auch moderne Therapieansätze, die eine Mobilisierung der Patienten anstreben, sind nicht durchführbar.

Bekannte Gasaustauscher, insbesondere Gasaustauscher, die für den Einsatz an einer Herzlungenmaschine (HLM) vorgesehen sind, sind zu voluminös und zu schwer, um beispielsweise von einem Patienten am Körper getragen werden zu können. Gegen einen derartigen mobilen Einsatz einer tragbaren Gasaustauschvorrichtung spräche dabei auch die bislang übliche orthogonale Orientierung der Schläuche und Konnektoren bei derartigen Gasaustauschern, was einen großen Raumbedarf des Gasaustauschers bedingt.

Die Druckschrift WO 2014/183852 A1 offenbart ein Oxygenatormodul mit einem Hohlfaserbündel zum Gasaustausch zwischen Blut und einem Gas mit einer Verpottung mit kreisförmiger Innenmantelfläche und einem Oxygenator, der ein solches Oxygenatormodul umfasst. In dem Oxygenator kann stromauf des Oxygenatormoduls eine Verteilereinrichtung, bspw. ein Tangentialverteiler, angeordnet sein.

### Kurze Beschreibung der Erfindung

Der vorliegenden Erfindung liegt die Aufgabe zugrunde, eine verbesserte Vorrichtung zur Behandlung einer biologischen Flüssigkeit bereitzustellen, welche wenigstens einen der Nachteile der bekannten Systeme löst und die einen platzsparenderen Gasaustauscher, insbesondere auch für eine mobile Anwendung des Gasaustauschers bereitstellt. Vornehmlich ist es eine Aufgabe der vorliegenden Erfindung, einen für einen Patienten tragbaren Gasaustauscher bereitzustellen. Es ist insbesondere eine Aufgabe der vorliegenden Erfindung, einen Platzbedarf eines Gasaustauschers zu reduzieren, so dass es ermöglicht ist, den Gasaustauscher, vorzugsweise direkt, an einem Patientenkörper zu transportieren.

Die Aufgabe der vorliegenden Erfindung wird durch eine Vorrichtung gemäß Anspruch 1 gelöst. Vorteilhafte Weiterbildungen der Erfindung sind Gegenstand der abhängigen Ansprüche.

Gemäß einem Aspekt der Erfindung wird eine Vorrichtung zur Behandlung einer biologischen Flüssigkeit bereitgestellt, die ein Gehäuse mit zumindest einer ersten, eine Kavität bildenden Kammer, sowie ein zumindest teilweise in der ersten Kammer angeordnetes Gasaustauschmittel aufweist. Das Gasaustauschmittel ist als ein durch eine Anordnung mehrerer übereinander geschichteter Fasermatten aus einer Mehrzahl von Hohlfasern gebildetes Faserbündel ausgebildet. Die erste Kammer ist zur Aufnahme einer zu behandelnden Flüssigkeit, hier insbesondere einer biologischen Flüssigkeit, wie beispielsweise Blut, bestimmt. An einer Oberfläche des Gehäuses, die im Folgenden auch als Einlassoberfläche bezeichnet wird, ist ein Einlassabschnitt zum Einlass der zu behandelnden Flüssigkeit in die erste Kammer ausgebildet. Der Einlassabschnitt ist dabei erfindungsgemäß in einem spitzen Winkel relativ zu der Einlassoberfläche des Gehäuses ausgebildet, an der der Einlassabschnitt ausgebildet ist. Erfindungsgemäß verläuft der Einlassabschnitt in einem spitzen Anströmwinkel (α) relativ zu einer Oberfläche des Faserbündels, die durch eine erste Fasermatte definiert ist und die sich im Wesentlichen parallel zu einer durch die Einlassoberfläche definierten Ebene erstreckt.

Insbesondere kann die erfindungsgemäße Vorrichtung als Gasaustauschmittel eine zweite, zumindest teilweise in der ersten Kammer ausgebildete Kammer aufweisen oder eine zweite Kammer bilden. Die zweite Kammer ist zur Aufnahme eines ersten Fluids, insbesondere eines ersten Gases bestimmt. Die zweite Kammer kann durch mindestens eine semi-permeable Membran von der ersten Kammer getrennt ausgebildet sein. Vorzugsweise ist die semi-permeable Membran eine gasdurchlässige und flüssigkeitsundurchlässige Membran. Die Membran kann beispielsweise einem Transfer von zumindest einer vorbestimmbaren Molekülart zwischen erster und zweiter Kammer dienen. Der Transfer kann dabei von der ersten Kammer zu der zweiten Kammer und/oder von der zweiten Kammer zu der ersten Kammer erfolgen. Es kann dabei auch eine Mehrzahl erster und/oder zweiter Kammern vorgesehen sein.

"Semi-permeabel" soll im Zusammenhang der Erfindung derart verstanden werden, dass die Wandung teilweise durchlässig ist. Eine Durchlässigkeit soll dabei für vorbestimmte Moleküle gegeben sein, insbesondere für spezielle Gasmoleküle wie beispielsweise Sauerstoff oder Kohlenstoffdioxid. Insofern beinhaltet der Begriff "semi-permeabel" auch eine selektive Permeabilität für spezifische, vorbestimmbare Moleküle oder Verbindungen.

Das Gasaustauschmittel, insbesondere die zweite Kammer, kann derart ausgebildet und angeordnet sein, dass das Gasaustauschmittel von der mindestens einen gasdurchlässigen und flüssigkeitsundurchlässigen Membran abgegrenzt bzw. umschlossen ist und derart, dass eine biologische Flüssigkeit, die in der ersten Kammer vorgesehen ist, das Gasaustauschmittel zumindest teilweise umgeben oder umströmen kann.

Soweit im Folgenden auf eine "zweite Kammer" Bezug genommen wird, dient dies lediglich zur erleichterten Verständlichkeit der vorliegenden Erfindung. Die zweite Kammer ist jedoch lediglich als Teil bestimmter speziellerer Ausführungsformen des Gasaustauschmittels zu verstehen. Das Gasaustauschmittel bezeichnet dabei alle diejenigen Strukturen, die im Sinne der Erfindung einen Gasaustausch zwischen einer zu behandelnden Flüssigkeit, wie beispielsweise einer biologischen Flüssigkeit wie Blut, und einem für den Gasaustausch notwendigen Fluid ermöglichen.

Die Membran zwischen der ersten Kammer und dem Gasaustauschmittel ist typischerweise halbdurchlässig oder semi-permeabel und besitzt normalerweise Poren, deren Größe die Funktion und den Effekt der jeweiligen Membran bestimmen. Mit anderen Worten kann jede Membran einerseits eine poröse Membran sein, d.h. eine Membran, die diskrete Poren aufweist. Andererseits kann die Membran eine homogene Löslichkeitsmembran ohne diskrete Poren sein, in der der Stofftransport durch Lösung des Permeats (z.B. des Gases) im Polymer erfolgt und die Trennung aufgrund unterschiedlicher Löslichkeiten im Polymer stattfindet. Vorzugsweise ist die Membran eine nicht-poröse permeable Membran. So kann ein Gasaustausch zwischen dem Fluid in dem Gasaustauschmittel und der biologischen Flüssigkeit in der ersten Kammer ermöglicht werden. Der Gasaustausch kann dem konvektiven und diffusiven Stoffaustausch unterliegen. Vorzugsweise ist der Gasaustausch diffusiv und wird über die Differenz der Gaskonzentration auf beiden Seiten der Membran bestimmt.

Die erfindungsgemäße Vorrichtung zur Behandlung einer biologischen Flüssigkeit kann durch die gewinkelte Anordnung des Einlassabschnitts relativ zu der Oberfläche des Gehäuses eine platzsparendere Einleitung der Flüssigkeit in die erste Kammer ermöglichen im Vergleich zu den üblicherweise in den bekannten stationären Systemen verwendeten orthogonalen Anschlüssen. Es kann erfindungsgemäß eine tangentiale Anströmung und Zu- und Ableitung der Flüssigkeit erfolgen, so dass Zuund Ableitungen in nahezu derselben Ebene geführt werden können, in der auch das Gehäuse bzw. die Gehäuseoberfläche ausgebildet ist. Dies kann insbesondere einen Transport nahe oder auch direkt am Körper, beispielsweise eines Patienten, ermöglichen. Dies kann eine Mobilität der Vorrichtung und folglich auch eines Verwenders erhöhen.

Es versteht sich dabei, dass alle mit der Flüssigkeit in Kontakt kommenden Komponenten entweder steril herstellbar oder sterilisierbar sind.

Vorzugsweise ist die erste Kammer als Durchflusskammer gestaltet und weist einen Einlass und einen von dem Einlass separaten Auslass auf. Vorzugsweise kann die zweite Kammer ebenso als Durchflusskammer ausgebildet sein. Die erste Kammer kann dabei bevorzugt zum Durchfluss in einer Richtung gegen oder quer zur Durchflussrichtung der zweiten Kammer bestimmt sein.

In einigen Ausführungsformen der Erfindung ist das Gasaustauschmittel, insbesondere die zweite Kammer, in mehrere Unterkammern unterteilt, sodass die Vorrichtung mehrere zweite Kammern umfasst, die zur Aufnahme eines Gases bestimmt und von der ersten Kammer durch eine gasdurchlässige und flüssigkeitsundurchlässige Membran getrennt sind. Die mehreren zweiten Kammern befinden sich innerhalb der ersten Kammer bzw. sind von der ersten Kammer im Wesentlichen umgeben. Vorzugsweise haben die zweiten Kammern einen länglichen und bevorzugt im Wesentlichen zylinderförmigen Aufbau, der im Querschnitt einen oder mehrere durchgängige Hohlräume aufweist. Eine den Querschnitt abgrenzende Wand der zweiten Kammern bildet zumindest teilweise die gasdurchlässige und flüssigkeitsundurchlässige Membran.

In einer Weiterbildung der Erfindung sind die mehreren zweiten Kammern in einer oder mehreren Reihen nebeneinander und bevorzugt auch in einem Abstand voneinander angeordnet. Ferner können die mehreren zweiten Kammern in mehreren Lagen angeordnet sein. Die zweiten Kammern sind als Hohlfasern ausgebildet, sodass die Wand jeder Hohlfaser die gasdurchlässige und flüssigkeitsundurchlässige Membran bildet. Der Abstand zwischen den mehreren, nebeneinander angeordneten zweiten Kammern liegt bevorzugt im Bereich von 50 µm bis 1cm, weiter bevorzugt im Bereich von 100 µm bis 1 mm, und noch weiter bevorzugt im Bereich von 100 µm bis 500 µm. Dieser Abstand kann beliebig gewählt bzw. eingestellt werden.

In vorteilhaften Weiterbildungen der vorliegenden Erfindung weist das Gehäuse des Weiteren einen Auslassabschnitt an einer Oberfläche, der Auslassoberfläche, des Gehäuses zum Auslassen der biologischen Flüssigkeit aus der Vorrichtung auf. Dabei kann der Auslassabschnitt ebenfalls in einem spitzen Winkel relativ zu der Oberfläche des Gehäuses angeordnet sein. Der Winkel, den der Auslassabschnitt gegen die Auslassoberfläche einnimmt, kann dabei insbesondere derselbe Winkel sein, wie derjenige, den der Einlassabschnitt gegen die entsprechende Einlassoberfläche einnimmt. Auf diese Weise kann ein Platzbedarf der Vorrichtung weiter reduziert werden, da eine bislang notwendige platzeinnehmende orthogonale Anordnung des Auslassanschlusses und somit eine entsprechende orthogonale Führung von Leitungen vermieden werden kann.

Es ist alternativ denkbar, dass der Winkel des Auslassabschnitts sich von dem Winkel des Einlassabschnitts gegen die entsprechende Oberfläche unterscheidet. Dies kann, gerade im Hinblick auf eine Mobilität eines Trägers, eine Berücksichtigung an die Anatomie des Trägers oder an die Position der Vorrichtung an einem Träger oder in einem Gehäuse oder einer Tragevorrichtung ermöglichen. Der Auslassabschnitt kann grundsätzlich auch an derselben Oberfläche des Gehäuses vorgesehen sein, wie der Einlassabschnitt.

Es versteht sich auch, dass die relative Position von Einlassabschnitt und Auslassabschnitt variieren kann. So können der Einlass- und der Auslassabschnitt beispielsweise waagerecht nebeneinander, oder senkrecht übereinander angeordnet sein. Auch ist sowohl eine parallele oder eine orthogonal Anordnung des Auslassabschnitts relativ zu dem Einlassabschnitt möglich. In einigen Ausführungsformen der vorliegenden Erfindung ist der Auslassabschnitt dem Einlassabschnitt diametral gegenüberliegend. Es ist auch denkbar, dass der Einlassabschnitt an einer Position angeordnet ist, die sich, in einer Position, in der die erfindungsgemäße Vorrichtung üblicherweise verwendet wird, an einem oberen Abschnitt der Gasaustauschvorrichtung befindet. Der Auslassabschnitt kann in diesem Fall insbesondere an einem unteren Abschnitt der Vorrichtung ausgebildet sein. Dadurch kann ein Durchfluss durch die Vorrichtung von dem Einlassabschnitt zu dem Auslassabschnitt erleichtert werden, insbesondere deshalb, da die Gravitationskraft den Fluss der Flüssigkeit durch die Vorrichtung unterstützen kann. Auf diese Weise kann ein Herz-KreislaufSystem eines Patienten, der die Gasaustauschvorrichtung trägt, entlastet werden.

In einigen Weiterbildungen der Erfindung ist der Winkel, den der Einlassabschnitt relativ zu der jeweiligen Einlassoberfläche, also der Einlasswinkel, und/oder der Winkel, den der Auslassabschnitt relativ zu der Auslassoberfläche des Gehäuses, also der Auslasswinkel, einnehmen, kleiner als 45°, vorzugsweise kleiner als 25°, insbesondere zwischen 15° - 20°.

Der Einlassabschnitt kann derart an der Oberfläche des Gehäuses ausgebildet sein, dass eine durch den Einlassabschnitt geleitete biologische Flüssigkeit so in die erste Kammer einleitbar ist, dass diese an einem zentralen Bereich des Gasaustauschmittels, insbesondere des Faserbündels, in die erste Kammer eingeleitet wird. Ein zentraler Bereich bezeichnet dabei einen Bereich des Gasaustauschmittels, bzw. des Faserbündels, der sich in einer lateralen Ausdehnung, also in denjenigen Richtungen, die im Wesentlichen eine zu der Einlassoberfläche parallele Oberfläche aufspannen, im Zentrum bzw. im Bereich des Zentrums des Faserbündels befindet.

Es versteht sich, dass auch die Zu- und/oder Ableitung für das erste Fluid oder weitere Komponenten in das Gehäuse tangential bzw. in einem flachen Winkel, beispielsweise relativ zu einer Umfangsfläche des Gehäuses, erfolgen kann. Auf diese Weise kann eine weitere Platzersparnis für die Gasaustauschvorrichtung ermöglicht werden.

Das Gasaustauschmittel, insbesondere die zweite Kammer bzw. die zweiten Kammern, ist als ein durch eine Mehrzahl von Hohlfasern gebildetes Faserbündel ausgebildet, das zumindest teilweise in der ersten Kammer angeordnet ist.

Die erste Kammer kann eine, im Wesentlichen, zylindrische Form aufweisen. Auf diese Weise wird eine Kavität gebildet, die im Wesentlichen eine Zylinderform aufweist. Das bedeutet, die Kavität weist in den Randbereichen keine Ecken oder unstete Randbereiche auf. Zusätzlich oder alternativ kann auch das Gasaustauschmittel, insbesondere das Faserbündel, eine, im Wesentlichen, zylindrische Form aufweisen. In einigen Ausführungsformen kann die Form des Gasaustauschmittels bzw. des Faserbündels und die Form der Kavität, also die Innenkontur der ersten Kammer, aufeinander abgestimmte Formen und Dimensionen aufweisen, so dass das Gasaustauschmittel möglichst ideal in der Kavität der ersten Kammer angeordnet sein kann. Eine ideale Anordnung in diesem Zusammenhang wäre eine Anordnung, in der ein größtmöglicher Bereich des Gasaustauschmittels gleichmäßig von der biologischen Flüssigkeit, vorzugsweise laminar und mit im Wesentlichen konstanter, gleichmäßiger Fließgeschwindigkeit über den gesamten Strömungsquerschnitt, umströmt wird.

Aus dem Stand der Technik sind dem Fachmann Vorrichtungen bekannt, die einen Gasaustauscher mit einer kubischen Kavität zum Durchfluss einer biologischen Flüssigkeit aufweisen. Durch das Anordnen des Auslassabschnitts und des Einlassabschnitts in einem spitzen Winkel zu der entsprechenden Oberfläche kann eine parallele oder zumindest annähernd parallele Anströmung der zweiten Kammern erfolgen. Die Verwendung einer zylindrischen Kavität entsprechend der oben dargestellten Weiterbildung der Erfindung gemäß einiger Ausführungsformen kann eine homogenere Verteilung der Strömungsgeschwindigkeit im Vergleich zu einer kubischen Kavität ermöglichen.

Die Verwendung eines zylindrischen Faserbündels kann zudem eine bessere Ausnutzung der Kavität, also des zum Gasaustausch zur Verfügung stehenden Raumes in der ersten Kammer ermöglichen. Dies kann die Zuverlässigkeit und Haltbarkeit der Vorrichtung, beispielsweise durch Reduzierung auftretender Verwirbelungen und sich daraus ergebende Degenerationen, verringern. Solche Degenerationen finden insbesondere in Eckbereichen einer kubischen Kavität statt, woraus bei Verwendung von Blut als Flüssigkeit in der ersten Kammer ein erhöhtes Koagulationsrisiko resultiert. Die Ausbildung einer zylindrischen Kavität, unter Vermeidung derartiger Eckbereiche kann dieses Risiko reduzieren. Zylindrische Faserbündel sind natürlich auch grundsätzlich zur Anströmung mittels orthogonal angeordneter Einlassabschnitte geeignet.

Der Gasaustauscher kann einen Druckmittler an einer dem Einlassabschnitt zugewandten Seite der ersten Kammer aufweisen. Der Druckmittler dient zum Ausgleichen von Druckunterschieden in einem Strömungsquerschnitt der biologischen Flüssigkeit. Der Strömungsquerschnitt der Flüssigkeit kennzeichnet dabei den gesamten Bereich, den die Flüssigkeit bzw. die Flüssigkeitsfront in der ersten Kammer okkupiert bzw. durchfließt. Insbesondere bei der Verwendung von biologischen Flüssigkeiten wie Blut, ist es wünschenswert, dass die Flüssigkeit gleichmäßig durch die Vorrichtung, vorliegend die erste Kammer der Vorrichtung, fließt, ohne dass sich Verwirbelungen in dem Flussprofil einstellen.

Anderenfalls können sich insbesondere Regionen ausbilden, in denen die Flussrate so stark reduziert ist, dass sich eine verstärkte Anlagerung von Flüssigkeitsbestandteilen an den Wandungen, vor allem auch an den Membranen zu dem Gasaustauschmittel, bilden. Dies kann zum einen die Funktion des Gasaustausches beeinträchtigen. Zum anderen kann dies zu Verunreinigung der Flüssigkeit führen, wenn sich abgelagertes Material wieder von den Wänden ablöst. Ein Druckmittler, der eine gleichmäßigere Verteilung des Anströmungsdruckes in die erste Kammer bewirken kann, kann diesen Effekt reduzieren. Somit kann ein derartiger Druckmittler die Zuverlässigkeit der erfindungsgemäßen Vorrichtung erhöhen.

Vorteilhafterweise kann der Druckmittler eine im Wesentlichen schräge Ebene aufweisen, entlang derer die biologische Flüssigkeit beim Einfließen in die Vorrichtung geleitet ist. Dies kann die Bildung von Druckunterschieden weiter reduzieren und Strömungsablösungen, also stationäre Wirbel, können besser vermieden werden.

In einigen Ausführungen der Erfindung weist zumindest eine der Oberflächen der Vorrichtung, also die Einlassoberfläche und/oder die Auslassoberfläche einen Deckel auf oder stellt als Ganzes einen Deckel dar. Ein derartiger Deckel kann insbesondere als ein abnehmbarer Deckel ausgebildet sein, so dass ein Zugriff auf die erste Kammer bzw. auf das Gasaustauschmittel, insbesondere auf die zweite Kammer, in der Vorrichtung ermöglicht ist. Der Deckel ist dabei grundsätzlich in einem Bereich stromaufwärts der ersten bzw. der zweiten Kammer angeordnet. Der Deckel kann auch mit der bzw. mit den zweiten Kammern verbunden sein und diese nach außen abschließen.

In vorteilhaften Ausführungsformen kann ein Druckmittler zumindest an dem Deckel der Einlassoberfläche auf dessen Innenseite, vorzugsweise in Form einer schrägen Ebene, vorgesehen sein. Auf diese Weise kann bereits beim Einlass der Flüssigkeit ein Druckunterschied, welcher durch die asymmetrische Anströmung verursacht wird, zumindest teilweise ausgeglichen werden. Auf diese Weise kann wiederum eine homogenere Druckverteilung und daraus folgend eine homogenere Verteilung der Strömungsgeschwindigkeiten erfolgen. Ein derartiger Druckmittler wird auch als Blutverteilerplatte bezeichnet.

In wiederum weiteren Weiterbildungen kann in der Vorrichtung ein Verteilermittel ausgebildet sein, das ausgebildet ist, die biologische Flüssigkeit in einer zur Strömungsrichtung im Wesentlichen lateralen Richtung zu verteilen. Auf diese Weise kann eine verbesserte Anströmung einer größeren Fläche des Gasaustauschmittels, insbesondere eines Faserbündels bzw. der zweiten oder weiteren Kammern, erreicht werden. So kann, durch eine möglichst optimierte Anströmung der gesamten verfügbaren Oberfläche des Gasaustauschmittels, die Effizienz der Vorrichtung erhöht werden. Dies kann aufgrund einer erhöhten Wirkungseffizienz weitere Miniaturisierung der Vorrichtung erlauben. Das Verteilermittel kann auch integral mit dem Druckmittler ausgebildet sein.

In vorteilhaften Weiterbildungen der Erfindung weist das Verteilermittel ausgehend von einer dem Einlassabschnitt zugewandten Seite einen kanalartigen Abschnitt auf, der sich in der Strömungsrichtung der biologischen Flüssigkeit in lateraler Richtung öffnet. Eine "laterale Richtung" stellt dabei eine Richtung dar, die sich im Wesentlichen in derselben Richtung erstreckt, wie die Einlassoberfläche, an der der Einlassabschnitt angeordnet bzw. durch die der Einlassabschnitt ausgebildet ist. Es versteht sich, dass zusätzlich zu der Aufweitung in der lateralen Richtung, der kanalartige Abschnitt eine Neigung aufweisen kann. Die Neigung kann insbesondere in eine Richtung vorgesehen sein, die dem Winkel des Einlassabschnitts zu der Einlassoberfläche entspricht. Alternativ kann der Neigungswinkel ein beliebiger von dem Einlasswinkel unterschiedlicher Winkel sein. Auf diese Weise kann das Verteilermittel gleichzeitig eine schräge Ebene darstellen, um die zu behandelnde Flüssigkeit verbessert in die erste Kammer und über das Gasaustauschmittel zu leiten. Vorzugsweise ist das Verteilermittel daher von dem Einlassabschnitt zu der ersten Kammer hin geneigt.

Das Verteilermittel kann einstückig mit dem Druckmittler und/oder mit dem Einlassabschnitt und/oder mit dem Deckel auf der Einlassseite und/oder mit der Einlassoberfläche des Gehäuses ausgebildet sein. Dadurch müssen weniger einzelne Komponenten in der erfindungsgemäßen Vorrichtung verbaut werden. Dies kann einen vereinfachten Herstellungs- und Wartungsprozess ermöglichen. Zudem kann, durch die konsequenterweise reduzierte Notwendigkeit für Abdichtungen zwischen einzelnen Komponenten, die Zuverlässigkeit der Vorrichtung erhöht werden.

Die oben ausgeführten Weiterbildungen können in wiederum weiteren Ausführungsformen auch auf der Auslassseite der Vorrichtung hinsichtlich des Auslassabschnitts realisiert werden. Derartige Modifizierungen des Auslassabschnitts können zu einem verbesserten Abfluss der Flüssigkeit aus der erfindungsgemäßen Vorrichtung führen. Dies kann insbesondere auch ermöglichen, dass, beispielsweise bei einer Verwendung der Vorrichtung als Gasaustauscher in Form einer künstlichen Lunge, eine von extern bereitgestellte Pumpleistung zur Aufrechterhaltung der Zirkulation der Flüssigkeit reduziert werden kann, oder dass auf eine externe Pumpe gänzlich verzichtet werden kann.

Neben der ersten Kammer kann in einer Weiterbildung der Erfindung in der ersten Kammer zudem eine dritte Kammer ausgebildet sein. Die dritte Kammer kann dabei auch als Teil des Gasaustauschmittels ausgebildet sein. Die dritte Kammer kann durch mindestens eine flüssigkeitsdurchlässige Membran von der ersten Kammer getrennt sein und einem Entzug einer oder mehrerer Komponenten der biologischen Flüssigkeit dienen. Dies kann der erfindungsgemäßen Vorrichtung weitere Einsatzgebiete eröffnen, in dem auch spezifische Flüssigkeitsbestandteile aus der biologischen Flüssigkeit entzogen oder auch der Flüssigkeit beigesetzt werden.

In alternativen Ausführungsformen kann die dritte Kammer auch analog zu der zweiten Kammer ausgebildet sein, jedoch in einer zu der zweiten Kammer abweichenden räumlichen Anordnung. So können beispielsweise die zweite und die dritte Kammer jeweils als eine Mehrzahl von Schichten eines Faserbündels aufweisen, die jeweils aus einer Mehrzahl an parallel angeordneten Hohlfasern bestehen. Die einzelnen Schichten der zweiten und dritten Kammern können dann abwechselnd gestapelt angeordnet sein, wobei die Hohlfasern einer Schicht nicht parallel zu den Hohlfasern einer angrenzenden Schicht ausgerichtet sind. Insbesondere können die Hohlfasern einer Schicht rechtwinklig zu der Erstreckungsrichtung der Hohlfasern einer angrenzenden Schicht ausgerichtet sein. Auf diese Weise kann die Kontaktfläche der biologischen Flüssigkeit mit den zweiten und dritten Kammern erhöht werden.

Es versteht sich, dass die oben indizierten Ausführungsformen auch in Kombination miteinander ausgeführt werden können, ohne sich von dem erfinderischen Gedanken zu entfernen, soweit dies nicht ausdrücklich ausgeschlossen ist.

Gemäß einem weiteren Aspekt der vorliegenden Erfindung kann die erfindungsgemäße Vorrichtung als ein Gasaustauscher ausgebildet sein, der in einer künstlichen Lunge oder in einem Bioreaktor verwendet werden kann.

Es ist somit grundsätzlich möglich, eine Vorrichtung zur Behandlung einer biologischen Flüssigkeit mit verschiedenen Anschlüssen und geometrisch verschieden ausgebildeten Gasaustauschelementen bereitzustellen. Ziel einer Vorrichtung zur Behandlung einer biologischen Flüssigkeit im Sinne der vorliegenden Erfindung ist es, eine biologische Flüssigkeit, insbesondere Blut, von Kohlenstoffdioxid oder anderen Gasen oder weiteren, auch komplexeren Molekülen abzureichern und/oder mit Sauerstoff oder anderen Gasen oder weiteren Molekülen anzureichern. Natürlich kann auch eine Abreicherung anderer Bestandteile der Flüssigkeit grundsätzlich erzielt werden.

Insbesondere bei der Behandlung von komplexen Flüssigkeiten, die unter anderem lebende Zellen enthalten, wie beispielsweise Blut, ist es vorteilhaft, jedweden externen Stress auf ein Minimum zu reduzieren. Dies beinhaltet beispielsweise auch, dass eine Anströmung einer Gasaustauschvorrichtung möglichst derart erfolgt, dass eine laminare, stetige Strömung der biologischen Flüssigkeit aufrechterhalten wird. Dabei sollen Verwirbelungen möglichst vermieden werden und vorteilhafterweise eine gleichmäßige, homogene Anströmung des Gasaustauschabschnitts bewirkt werden. Vorzugsweise kann, insbesondere in einem Fall, in dem die biologische Flüssigkeit Blut ist, eine Anströmung mit physiologischer Strömungsgeschwindigkeit der Flüssigkeit erfolgen. Dies kann, neben der oben dargelegten Form, gemäß der bevorzugten Ausführungsformen der Erfindung natürlich auch auf weitere Weisen erfolgen. So ist es bereits vorteilhaft, wenn die Anströmung des Gasaustauschmittels, insbesondere einer Fasermatte, eine kontinuierliche Strömung erlaubt.

Im Sinne der Erfindung wird hier und im Folgenden der Begriff "Fasermatte" auch stellvertretend für ein Gasaustauschmittel verwendet. Mit diesem Begriff "Fasermatte" werden ein oder mehrschichtige Strukturen bezeichnet, die aus Hohlfasern gebildet sind, die in einer mattenartigen Anordnung ausgebildet werden. Übereinander geschichtete Faserschichten, bzw. individuelle Fasermatten, können jeweils um einen Winkel verdreht zu einer darunterliegenden Schicht angeordnet werden, wodurch eine runde Fasermattenanordnung bzw. ein rundes Faserbündel geschaffen werden kann. Abhängig insbesondere von Einsatzzweck und avisierter Größe können Fasermatten generell aus mehreren einzelnen Faserschichten, also individuellen Fasermatten, aufgebaut sein oder lediglich aus einer einzelnen Schicht bestehen.

Es sind aus dem Stand der Technik derartige Fasermatten bekannt, die insbesondere eine rechteckige Grundform aufweisen, und sich in Form einer Fasermattenschichtanordnung in einer Höhenrichtung um eine bestimmte Höhe erstrecken können. Derartige Fasermattenanordnungen oder Faserbündel weisen insofern eine quaderartige Form auf. Die Problematik mit solchen quaderförmigen Fasermattenanordnungen kann dabei jedoch darin liegen, dass, wenn man von einer korrespondierenden Form der Kavität in der Gasaustauschvorrichtung ausgeht, also einer ebenfalls quaderförmigen Kavität, eine zumindest weitgehend gleichmäßige Strömung nicht gewährleistet werden kann. Insbesondere entlang der Kanten und in Eckabschnitten der Kavität kann es verstärkt zu Verwirbelungen der Flüssigkeit kommen, was zu einer Degradierung der Flüssigkeit führen kann. Die Effizienz des Gasaustausches in diesen Randbereichen kann somit reduziert sein.

Es ist jedoch gerade ein Bestreben, eine möglichst hohe Gasaustauschrate, eine Vermeidung einer Degradierung der Flüssigkeit und/oder eine möglichst effiziente Ausnutzung des Gasaustauschelements zu erlangen.

Dabei ist es zum einen denkbar, ein in der Grundform rundes Gasaustauschmittel, vorzugsweise ein kreisförmiges Gasaustauschmittel, in einer derartigen rechtwinkeligen Kavität anzuordnen. Auf diese Weise können Randeffekte in einer quaderförmigen Kavität die Gasaustauschrate in dem Gasaustauschmittel weniger oder sogar gar nicht beeinflussen.

Zudem ist es möglich, eine runde, vorzugsweise eine kreisrunde, Kavität vorzusehen, in der ein quaderförmiges oder ein rundes Gasaustauschmittel, insbesondere eine Fasermatte oder eine Fasermattenanordnung, angeordnet wird. Die Kontur des Gasaustauschmittels korrespondiert dabei idealerweise mit der Form der Kavität, so dass ein möglichst großes Volumen der Kavität mit dem Gasaustauschmittel erfüllt werden kann. Dies kann das Volumen in der Kavität, das zum Gasaustausch zur Verfügung steht, idealerweise vergrößern.

Die Verwendung einer quaderförmigen Fasermatte oder Fasermattenanordnungen in einer runden Kavität kann es erlauben, Randeffekte an der Wand der Kavität, wo die Fließgeschwindigkeit der Flüssigkeit zwangsläufig minimal ist, zu vermeiden. So kann eine Verbesserung der Effizienz des Gasaustauschelements, also hier der Fasermatte oder Fasermattenanordnung, erreicht werden.

Die Form der Kavität beispielsweise zur Aufnahme der Fasermatte bzw. Fasermattenanordnung in der Vorrichtung wie auch die Form der Fasermatte oder der Fasermattenanordnung selbst kann dabei rund oder sogar kreisrund sein. Eine "runde" Form der Fasermatte bzw. der Fasermattenanordnung oder der Kavität kann jeder Form entsprechen, die einen stetigen Randverlauf, also einen Randverlauf ohne die Ausbildung von Kanten und Ecken, aufweist. Insbesondere kann eine runde Form auch eine elliptische Ausbildung beinhalten. Zudem kann eine runde Form auch weitere symmetrische oder unsymmetrische Formen umfassen, beispielsweise derartige Formen, die eine verbesserte Umströmung der Fasermatte oder der Fasermattenanordnung, allgemein also des Gasaustauschmittels, erlauben. Zudem kann unter dem Begriff "runde Form" auch beispielsweise eine Kavität verstanden werden, die abgerundete Ecken und Kanten aufweist. Die Form des Gasaustauschmittels kann dabei beispielsweise auch in einer Höhenrichtung entlang eines Querschnitts des Gasaustauschmittels variieren.

Im Übrigen sind Mischformen für die Form des Gasaustauschmittels denkbar, mit einer oder mehreren Ecken und im Übrigen runden bzw. abgerundeten Abschnitten. Grundsätzlich sind die Formen des Gasaustauschmittels also nicht beschränkt und die Gesamtheit der Formen kann in einer Kavität einer Gasaustauschvorrichtung angeordnet werden, so lange die notwendigen Anschlüsse vorliegen und die spezifischen Abmessungen der Kavität und des Gasaustauschmittels ein Einsetzen des Gasaustauschmittels in die Kavität erlauben. Die Formen der Kavität der ersten Kammer und des Gasaustauschmittels können dabei auch voneinander variieren.

Vorzugsweise weist das Gehäuse eine prismatische Form auf, bei der die Seitenflächen des Gehäuses als Prismenflächen ausgebildet sind, wobei der Habitus des Gehäuses gedrungen, insbesondere flach ist und wobei ein Bluteinlass an einer als Einlassoberfläche ausgebildeten Stirnfläche des prismatischen Gehäuses angeordnet ist.

Alternativ vorteilhaft umfasst die Vorrichtung zur Behandlung einer biologischen Flüssigkeit, ein insbesondere prismatisches Gehäuse mit einer ersten, eine Kavität bildenden Kammer, die zur Aufnahme der zu behandelnden Flüssigkeit ausgebildet ist, und wenigstens ein Gasaustauschmittel, das zumindest teilweise in der ersten Kammer angeordnet ist, wobei in einer Oberfläche des Gehäuses, insbesondere einem Deckel des Gehäuses, ein Einlassabschnitt zum Einlass der zu behandelnden Flüssigkeit in die erste Kammer ausgebildet ist. Der Einlassabschnitt verläuft dabei in einem spitzen Anströmwinkel relativ zu der Oberfläche des Faserbündels.

Günstig ist es, wenn der Anströmwinkel kleiner als 45° ist, vorzugsweise kleiner als 25°, besonders bevorzugt einen Wert von 5° bis 20° aufweist und insbesondere bei 15° liegt.

Weiterhin vorteilhaft ist es, wenn zu beiden Seiten des kanalartigen Abschnitts Strömungs-Leitflächen angeordnet sind, die ein gegenläufiges Gefälle zu dem Gefälle des kanalartigen Abschnitts aufweisen, so dass sich eine durch den kanalartigen Abschnitt definierte erste Achse und eine durch die Strömungs-Leitflächen und definierte zweite Achse in einem spitzen Winkel von vorzugsweise 5° bis 20° schneiden. Durch die beiden Strömungs-Leitflächen und wird eine wirbelfreie Aufteilung des einströmenden Fluidstromes erreicht. In dem Fall, dass das eingeleitete Fluid Blut ist, wird dadurch eine Blutgerinnung (Verklumpung) und eine Blutschädigung (Hämolyse) effektiv verhindert. Es wird eine sehr gute dauerhafte Gasaustauscheffektivität bezogen auf Gasaustauschfläche erreicht.

Die Herstellung von derartigen runden, abgerundeten bzw. im Querschnitt inhomogenen Fasermatten und Fasermattenanordnung kann dabei auf ähnliche Weise erfolgen, wie dies für die Herstellung von quaderförmigen Fasermatten bekannt ist.

### Kurze Beschreibung der Zeichnungen

Die Erfindung sowie vorteilhafte Weiterbildungen davon werden nun anhand bestimmter in den beigefügten Zeichnungen dargestellter Ausführungsbeispiele erläutert, in denen gleiche Merkmale mit den gleichen Bezugszeichen versehen sind. Es zeigt:
- Figur 1: eine schematische Darstellung eines Ausschnitts eines Faserbündels für eine Vorrichtung gemäß einer Ausführungsform der Erfindung,
- Figur 2: eine schematische Darstellung einer erfindungsgemäßen Vorrichtung gemäß einer Ausführungsform der Erfindung,
- Figur 3: eine vergrößerte Querschnittansicht eines Konnektors für eine Vorrichtung gemäß einer Ausführungsform der Erfindung,
- Figur 4: eine schematische Darstellung eines Deckels für eine Vorrichtung gemäß einer Ausführungsform der Erfindung,
- Figur 5: eine schematische Darstellung eines weiteren Deckels für eine erfindungsgemäße Vorrichtung gemäß einer weiteren Ausführungsform der Erfindung,
- Figur 6: eine Querschnittansicht des Deckels aus Fig. 5 in einem Schnitt entlang der Linie VI-VI in Fig. 5,
- Figur 7: eine perspektivische Querschnittansicht des Deckels aus Fig. 5 in einem Schnitt entlang der Linie VII-VII in Fig. 5.

### Beschreibung der Ausführungsbeispiele der Erfindung

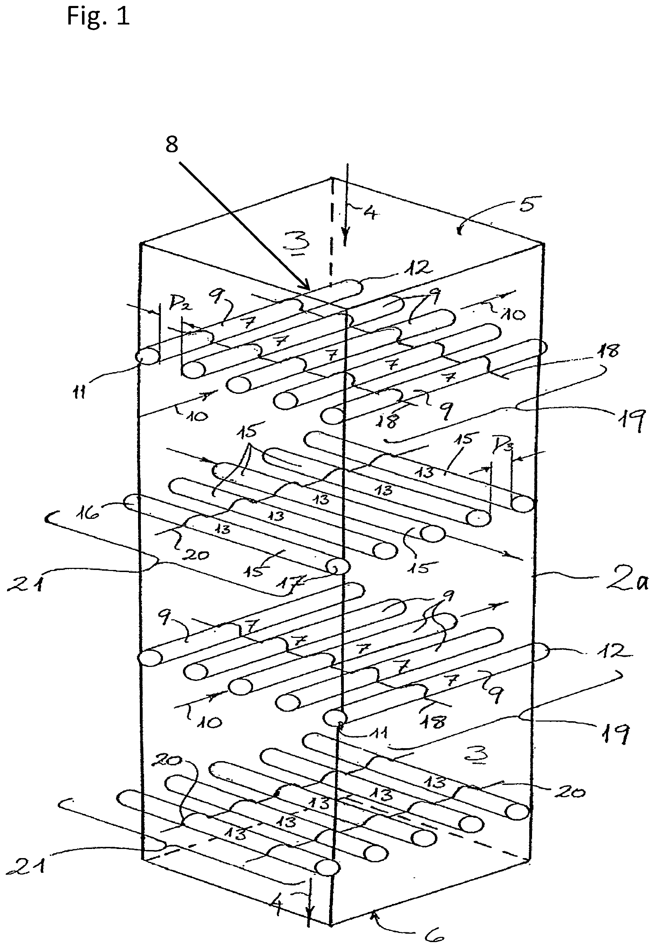

Figur 1 zeigt zum besseren Verständnis der vorliegenden Erfindung ein Beispiel für den Aufbau eines Gasaustauschmittels, hier in der Form eines mehrschichtigen Faserbündels aufgebaut aus zwei Schichtarten in Form von unterschiedlich orientierten Fasermatten 19 und 21. Dabei zeigt Figur 1 zum Grundverständnis der vorliegenden Erfindung lediglich eine schematische Ansicht des Faserbündels 18 mit den Fasermatten 19, 21 in einer Art Explosionsdarstellung der Schichten. Figur 1 lässt aufgrund des schematischen Charakters eines Teilbereichs des Faserbündels 18 keine Rückschlüsse zu auf die tatsächliche Form eines Faserbündels, wie es in einer erfindungsgemäßen Vorrichtung verwendet werden soll, insbesondere nicht hinsichtlich der Form, Proportionen oder Ausbildung von Faserbündelschichten oder deren spezifischer Eigenschaften. In einem zusammengeschlossenen Aufbau des Faserbündels wird aus den individuellen Faserschichten, auch als Fasermatten bezeichnet, eine Anordnung von Fasermatten, die zur Beschreibung vorliegend auch als Faserbündel bezeichnet wird. Das Faserbündel 18 weist eine durch eine erste Fasermatte 19, 21 definierte Oberfläche 8 auf.

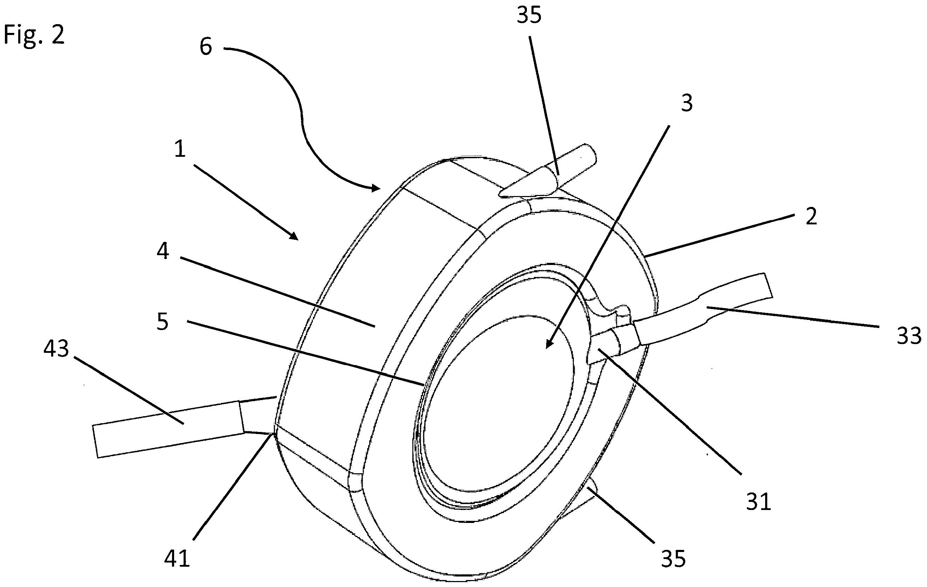

Eine Vorrichtung 1 gemäß der Erfindung, wie sie zudem auch in Figur 2 dargestellt ist, weist ein Gehäuse 2 auf, das eine erste Kammer 3 umschließt bzw. teilweise definiert. Die erste Kammer 3 ist zur Aufnahme einer biologischen Flüssigkeit, wie z.B. Blut, bestimmt und ist in der dargestellten Ausführungsform als eine Durchflusskammer ausgebildet. Die biologische Flüssigkeit bzw. das Blut fließt in die von den Pfeilen 4 gezeigte Richtung durch eine Einlassoberfläche 5 im Gehäuse 2 in die erste Kammer 3 ein und verlässt die erste Kammer 3 durch eine gegenüberliegende Auslassoberfläche 6. Das Gehäuse 2 besteht vorzugsweise aus einem für die biologische Flüssigkeit chemisch nicht reagierenden Kunststoff, wie z.B. Polyethylen oder Polyurethan. Das Gehäuse 2 weist eine prismatische Form auf, bei der die Seitenflächen als Prismenflächen 4 ausgebildet sind, die Basisfläche als Auslassoberfläche 6 und die Stirnfläche als Einlassoberfläche 5. Der Habitus des Gehäuses 2 ist dabei flach bzw. gedrungen, d.h. die Höhe des Gehäuses 2 zwischen der Auslassoberfläche 6 und der Einlassoberfläche 5 ist kleiner als seine seitliche Erstreckung quer zu seiner Höhe. Die Oberfläche 8 des Faserbündels 18 erstreckt sich im Wesentlichen parallel zu einer durch die Einlassoberfläche 5 definierten Ebene.

Ferner weist die Vorrichtung 1 eine rohrförmige zweite Kammer 7 auf, die sich durch die erste Kammer 3 erstreckt und von der ersten Kammer 3 im Wesentlichen umgeben ist. Die zweite Kammer 7 ist in Figur 2 nicht erkennbar. Eine rohrförmige Wand, begrenzt bzw. umschließt den Hohlraum der zweiten Kammer 7. Diese Wand ist relativ dünn und besteht bevorzugt aus einem Kunststoff und dient als Trägermaterial für eine äußere Schicht, welche zusammen mit der Wand eine gasdurchlässige und flüssigkeitsundurchlässige Membran 9 bildet. Die zweite Kammer 7 ist dazu vorgesehen, ein Fluid zu enthalten und zu leiten. Das Fluid ist in bevorzugten Ausführungsformen ein Gas, beispielsweise Umgebungsluft, Pressluft, ein mit Sauerstoff angereichertes Gasgemisch oder hochangereicherter oder reiner Sauerstoff. Die rohrförmige Wand ermöglicht dabei einen Transfer von Gasmolekülen zwischen der ersten Kammer 3 und der zweiten Kammer 7. Mit anderen Worten bildet die Membran 9 eine Trenn- bzw. Berührungsfläche, an der ein intimer Kontakt zwischen den molekularen Komponenten des Blutes und des in der zweiten Kammer enthaltenen Mediums zustande kommen bzw. stattfinden kann.

Die zweite Kammer 7 kann, wie in dem in Figur 1 gezeigten Fall, als eine Durchflusskammer ausgebildet sein. Die gasdurchlässige und flüssigkeitsundurchlässige Membran 9 ist bevorzugt selektiv für Kohlenstoffdioxid und/oder Sauerstoff durchlässig. In besonderen Ausführungsformen wird die beschriebene Erfindung verwendet, eine Oxygenierung, also eine Sauerstoffanreicherung, in der biologischen Flüssigkeit zu bewirken. Es ist jedoch alternativ auch denkbar, dass, abhängig von der spezifischen Anwendung, auch lediglich eine Kohlenstoffdioxid-Reduzierung, also eine Decarboxylierung, erfolgen soll. Abhängig von der gewünschten Anwendung wird folglich ein geeignetes Gas gewählt, beispielsweise mit Sauerstoff angereicherte Luft oder Kohlenstoffdioxid-arme Luft, um einen Gasaustausch mit der biologischen Flüssigkeit zu bewirken.

Das gewählte Gas fließt in die durch den Pfeil 10 in Figur 1 gezeigte Richtung durch einen Einlass 11 in die zweite Kammer 7 ein und verlässt die zweite Kammer 7 durch einen gegenüberliegenden Auslass 12. Das Gas, das durch die zweite Kammer 7 fließt, kann dabei zum Teil durch die gasdurchlässige und flüssigkeitsundurchlässige Membran 9 in die durch die erste Kammer 3 fließende Flüssigkeit übergehen. Zudem kann ein Gasanteil, der in der Flüssigkeit in der Kammer 3 gelöst ist, durch die Membran 9 in die zweite Kammer 7 übertreten. Hierdurch kann, im Falle dass die biologische Flüssigkeit Blut ist, eine Anreicherung des Blutes mit Sauerstoff oder eine Abreicherung des Blutes von Kohlenstoffdioxid stattfinden. Auf diese Weise erfolgt ein sogenanntes Ventilations- bzw. Lungenersatzverfahren an der Membran 9.

Ein Druck P₁ und/oder die Strömung der biologischen Flüssigkeit bzw. des Blutes in der ersten Kammer 3 kann im Verhältnis zum Druck P2 und/oder zur Strömung des durch die zweite Kammer 7 fließenden Sauerstoffes gewählt bzw. eingestellt werden. Auf diese Weise kann ein gewünschter Transfer von Sauerstoff in die Flüssigkeit und/oder Kohlenstoffdioxid aus der Flüssigkeit erreicht werden. Grundsätzlich gilt dabei, dass ein Gasaustausch von der Seite der beiden Kammern 3, 7 zu der jeweiligen anderen Kammer 7, 3 erfolgt, in der ein höherer Partialdruck des betreffenden Gases vorherrscht. Das Blut kann dazu z.B. mit einer Pumpe (nicht gezeigt) durch die erste Kammer 3 befördert werden oder es kann auch lediglich unter dem Druck des Kreislaufsystems des Patienten durch die erste Kammer 3 fließen. Es können auch alternativ oder zusätzlich Vorkehrungen getroffen werden, die eine druckunabhängige Anreicherung einer gewählten Komponente in einer der Kammern 3, 7 beeinflussen, beispielsweise die reversible oder irreversible Bindung von Gasmolekülen an entsprechend eingesetzten Oberflächen oder Bestandteilen der betreffenden Fluide, also der biologischen Flüssigkeit oder des Gases bzw. der Gase.

Die Vorrichtung 1 kann in besonderen Ausführungsformen ferner eine rohrförmige dritte Kammer 13 aufweisen, die sich ebenso wie die zweite Kammer durch die erste Kammer 3 erstreckt und von der ersten Kammer 3 im Wesentlichen umgeben ist. Eine rohrförmige Wand 14, die den Hohlraum der dritten Kammer 13 umschließt, ist relativ dünn und besteht bevorzugt aus einem Kunststoff. Wie mit der zweiten Kammer 7, dient die Wand 14 als Trägermaterial für eine äußere Schicht. Es ist dabei denkbar, dass die dritte Kammer eine von der zweiten Kammer 7 unabhängige Kammer ist, die eine gasdurchlässige, flüssigkeitsundurchlässige Membran 15 aufweist, die für dieselben oder für andere oder zusätzliche Gase permeabel ist wie die Membran 9 zwischen der ersten Kammer 3 und der zweiten Kammer 7. So kann beispielsweise eine zweite unabhängige Gasversorgung mit der Vorrichtung gekoppelt werden, oder, im Bedarfsfall, zusätzliche Gase in die Vorrichtung eingespeist werden oder eine Abreicherungsrate von Gasen aus der biologischen Flüssigkeit erhöht werden.

In besonderen Ausführungsformen kann die dritte Kammer 13 zusammen mit der Wand 14 auch eine flüssigkeitsdurchlässige Membran 15 bilden, sodass die rohrförmige Wand 14 einen Transfer von flüssigen Komponenten zwischen der ersten Kammer 3 und der dritten Kammer 13 ermöglicht. Insbesondere bildet diese Membran 15 eine Trenn- bzw. Berührungsfläche, welche einem Entzug einer oder mehrerer flüssiger Komponenten der biologischen Flüssigkeit dient. Es sind natürlich auch Ausführungsformen denkbar, in denen die Wandung zwischen der ersten Kammer 3 und der zweiten Kammer 7 eine flüssigkeitsdurchlässige Wand 9 darstellt.

Die flüssigkeitsdurchlässige Membran 15, die sich zwischen den ersten und dritten Kammern befindet, kann dann als Filter wirken, über den kleinere Moleküle wie Wasser von einer biologischen Flüssigkeit beispielsweise Blut abgepresst und größere Moleküle wie Eiweiße und Blutzellen zurückgehalten werden.

Es versteht sich, dass das Gasaustauschmittel der erfindungsgemäßen Vorrichtung eine oder mehrere zweite Kammern aufweisen kann. Es versteht sich ferner, dass der in Figur 1 gezeigte Aufbau eines Gasaustauschmittels in Form eines oder mehrerer Faserbündel zwar vorteilhaft für die Erfindung bzw. für Weiterbildungen der Erfindung sein kann. Jedoch sind auch Gasaustauschmittel mit anderer Schichtstruktur im Rahmen der Erfindung denkbar. Insofern stellt die Darstellung in Figur 1 lediglich ein beispielhaftes Gasaustauschmittel in Form eines Faserbündels für bestimmte Ausführungsformen der vorliegenden Erfindung dar.

In einer Ausführungsform der vorliegenden Erfindung, wie sie in der Figur 1 gezeigt ist, weist die zweite Kammer 7 bzw. die dritte Kammer 13 eine Mehrzahl von rohrförmigen Hohlfasern auf, wie im Folgenden erläutert wird. Das Gehäuse 2 umschließt bzw. definiert dabei zumindest teilweise die erste Kammer 3. Die erste Kammer 3 ist zur Aufnahme einer biologischen Flüssigkeit, wie beispielsweise Blut, bestimmt und ist als Durchflusskammer ausgebildet. Die erste Kammer 3 kann auch von einer in das Gehäuse eingesetzten, der Öffnung in dem Gehäuse entsprechenden Aufnahme zur Aufnahme der zweiten und/oder dritten Kammer entsprechen.

Es ist zu beachten, dass die erste Kammer 3 und die zweite Kammer 7 oder weitere Kammern 13 als ein zusammengeschlossenes, steril abgeschlossenes System bereitgestellt werden. In diesem Fall würde ein Gehäuse 2a, der ersten Kammer in eine entsprechende Öffnung des Gehäuses 2 der Gasaustauschvorrichtung eingesetzt werden. Dieser Fall ist in Figur 2 gezeigt. Alternativ kann ein Faserbündel auch in das Gehäuse 2 der Gasaustauschvorrichtung 1 auch eingesetzt werden, wobei die Innenwandung einer Öffnung der Gasaustauschvorrichtung 1 oder die Innenwandung eines Einsatzes in diese Öffnung die erste Kammer 3 definiert.

Die Vorrichtung 1 wie in Figur 1 gezeigt weist eine Mehrzahl rohrförmiger zweiter Kammern 7 auf, die nebeneinander in Reihen angeordnet sind. Die zweiten Kammern 7, also das Gasaustauschmittel, erstrecken sich parallel durch die erste Kammer 3 und sind von der ersten Kammer 3 im Wesentlichen umgeben. Die rohrförmigen Wände, die die Hohlräume der zweiten Kammern 7 umschließen, können in Form von Hohlfasern aus Polymethylpenten (PMP, auch unter dem Markennamen TPX^{®} bekannt), z.B. geschäumtes TPX, ausgebildet sein. In anderen Ausführungsformen können auf gleiche Weise andere Materialien verwendet werden. Die Wände der zweiten Kammern 7 und ihre äußeren Flächen bzw. Schichten bilden gasdurchlässige und flüssigkeitsundurchlässige Membranen 9, sodass ein Transfer von Gasmolekülen zwischen der ersten Kammer 3 und den sich im Inneren der Hohlfasern befindlichen zweiten Kammern 7 ermöglicht ist.

Die zweiten Kammern 7 sind zur Aufnahme eines Gases wie z.B. Sauerstoff oder Umgebungsluft bestimmt und sind ebenfalls als Durchflusskammern ausgebildet. Die gasdurchlässigen und flüssigkeitsundurchlässigen Membranen 9 können dabei selektiv für Sauerstoff und/oder Kohlenstoffdioxid durchlässig ausgebildet sein. Das gewählte Gas fließt in der in Figur 1 gezeigten Vorrichtung in die von dem Pfeil 10 gezeigte Richtung durch Einlässe 11 in die zweiten Kammern 7 ein und verlässt die zweiten Kammern 7 durch gegenüberliegende Auslässe 12. Wie vorher kann ein Teil des Sauerstoffes, der durch die zweiten Kammern 7 fließt, durch die gasdurchlässigen und flüssigkeitsundurchlässigen Membranen 9 in die durch die erste Kammer 3 fließende Flüssigkeit übergehen. Dies kann zu einer Anreicherung der Flüssigkeit mit Sauerstoff führen. Auf ähnliche Weise kann ein Transfer bzw. eine Entfernung von Kohlenstoffdioxid aus der Flüssigkeit, insbesondere Blut, durch die Membranen 9 in die zweiten Kammern 7 stattfinden, sodass ein sogenanntes Ventilations- bzw. Lungenersatzverfahren erfolgt.

Die in einer Reihe/Ebene nebeneinander angeordneten, die zweiten Kammern 7 bildenden Hohlfasern, beispielsweise TPX-Fasern, werden in einem textiltechnischen Verfahren mit Kettfäden 18 miteinander verbunden. Daraus entsteht eine Art Fasermatte 19 mit definierten Abständen D₂ zwischen den Fasern. Dieser Abstand D₂ zwischen den Fasern dient dazu, dass das durch die erste Kammer 3 fließende Blut durch die Matte 19 durchfließen und damit einen maximalen Kontakt mit der Berührungsflächen der Membranen 9 erreichen kann. In diesem Ausführungsbeispiel haben die einzelnen Hohlfasern einen äußeren Durchmesser im Bereich von 100 µm) bis 1 mm, bevorzugt im Bereich von 200 µm bis 600 µm, (z.B. etwa 400 µm), und sie sind in jeder Reihe bzw. Ebene nebeneinander mit einem Abstand D₂ im Bereich von 100 µm bis 500 µm angeordnet. Dieser Abstand kann beliebig gewählt werden. Daher wird klar, dass eine Vielzahl von Fasern nebeneinander gelegt und zu Matten 19 verarbeitet werden können. Die Abmessungen der Faser-Membranmatte 19 betragen bspw. etwa 10 cm x 15 cm.

In einigen Ausführungen der Erfindung haben die Fasermatten, im Folgenden auch bezeichnet als Faser-Membranplatten, eine runde Form, um mit einer zylindrischen Kavität in dem Gehäuse zu korrespondieren. Insbesondere sind diese Faser-Membranplatten Anordnungen mehrerer übereinander geschichteter Fasermatten. Natürlich ist es auch denkbar, dass quaderförmige oder kubische Faser-Membranplatten mit einer zylinderförmigen Kavität verwendet werden, oder dass runde, bzw. im Wesentlichen zylinderförmige, Faser-Membranplatten mit einer kubischen oder quaderförmigen Kavität der erfindungsgemäßen Vorrichtung verwendet werden.

Sind die Hohlfasern zu Matten bzw. Fasermatten 19, 21 verarbeitet, können diese Matten 19 anschließend weiterverarbeitet werden, in dem sie übereinander gestapelt werden. In Figur 1 werden lediglich zwei Lagen oder Matten 19 der zweiten, parallel verlaufenden Kammern 7 gezeigt und diese sind übereinander gestapelt. Es wird jedoch vom Fachmann verstanden, dass eine Vielzahl von solchen Matten 19 übereinander in der ersten Kammer 3 vorgesehen werden können. Obwohl in Figur 1 nicht so gezeigt, sind die Enden der die zweiten Kammern 7 bildenden Fasern zusammengebündelt bzw. zusammengeschaltet. Auf diese Weise können die einzelnen Einlässe über einen gemeinsamen Einlass 11 mit Gas, beispielsweise Umgebungsluft oder Sauerstoff, gespeist werden. Analog gehen die einzelnen Auslässe in einen gemeinsamen Auslass 12 über. Vorzugsweise gilt dies nicht nur für die zweiten Kammern 7 der einzelnen Matten 19 sondern für alle zweiten Kammern 7 in allen Matten 19. Diese sogenannte "Zusammenschaltung" von den Fasern der gleichen Orientierung erfolgt vorzugsweise über ein Vergussverfahren, z.B. mit Polyurethan. Hier werden im äußeren Bereich des Faserstapels die Enden der Fasern mit flüssigem Kunststoff umgossen. Nach Aushärtung des Kunststoffs wird dann von außen her scheibchenweise abgeschnitten, bis der Innenraum der Fasern eröffnet ist. Somit erreicht man eine gemeinsame Fluidzufuhr in die einzelnen Fasern bzw. Kammern.

Zudem weist die Vorrichtung 1 in Figur 1 mehrere rohrförmige dritte Kammern 13 auf, die sich ebenso wie die zweiten Kammern 7 parallel durch die erste Kammer 3 erstrecken und von der ersten Kammer 3 im Wesentlichen umgeben sind. Während in einigen Ausführungsformen die Hohlfasern genauso ausgebildet sein können, wie die oben beschriebenen Hohlfasern der Matten 19, ist es in besonderen Ausführungsformen auch möglich, dass die rohrförmigen Wände 14, die die Hohlräume der dritten Kammern 13 umschließen, in Form von Hohlfasern aus Polyethersulfon (PES) ausgebildet sind. In diesem Fall bilden die Wände 14 der Hohlfasern mit deren äußeren Flächen flüssigkeitsdurchlässige Membranen 15, welche einen Transfer von flüssigen Komponenten zwischen der ersten Kammer 3 und den sich im Inneren der Hohlfasern befindlichen, dritten Kammern 13 ermöglichen können. Insbesondere können die Membranen 15 Trenn- bzw. Berührungsflächen, welche einem Entzug einer oder mehrerer Komponenten der biologischen Flüssigkeit dienen, bilden.

Wie für die Hohlfasern der ersten Matten 19, werden die in einer Reihe bzw. Ebene nebeneinander angeordneten, die dritten Kammern 13 bildenden Hohlfasern, hier PES-Fasern oder TPX-Fasern, in einem textiltechnischen Verfahren mit Kettfäden 20 miteinander verbunden. Daraus entsteht wiederum eine Art Fasermatte 21 mit definierten Abständen D₃ zwischen den Fasern. Dieser Abstand D₃ zwischen den Fasern dient auch dazu, dass die durch die erste Kammer 3 fließende Flüssigkeit, insbesondere Blut, durch die Matte 21 hindurchfließen und damit einen maximalen Kontakt mit der Oberfläche der Membranen 15 erreichen kann. In Figur 1 werden lediglich zwei Lagen bzw. Matten 21 von dritten Kammern 13 gezeigt und diese sind als übereinander gestapelte Lagen 21 dargestellt. Wie mit den zweiten Kammern 7 haben die einzelnen Fasern in diesem Ausführungsbeispiel einen äußeren Durchmesser im Bereich von 100 µm bis 1 mm, bevorzugt im Bereich von 200 µm bis 600 µm, und sie sind in jeder Reihe bzw. Ebene nebeneinander mit einem Abstand D₃ vorzugsweise im Bereich von 100 µm bis 500 µm angeordnet. Eine Vielzahl von Fasern können daher nebeneinander gelegt und zu Matten 21 verarbeitet werden. Die Abmessungen jeder Faser-Membranmatte 21 betragen ebenfalls etwa 10 cm x 15 cm, wobei auch analog zu obigen Ausführungen für derartige mehrschichtige Faser-Membranmatten 21 verschiedene, insbesondere runde, Formen möglich sind.

Die Verarbeitung der einzelnen Faser-Membranmatten 21 erfolgt im Übrigen wie für die Faser-Membranmatten 19, wie oben detailliert beschrieben. Derartige Fasern bzw. Fasermatten sind beispielsweise aus der WO 2010/091867 grundsätzlich bekannt.

Die längliche Ausrichtung der vorzugsweise parallel angeordneten zweiten Kammern 7 verläuft rechtwinkelig zu der länglichen Ausrichtung der parallel angeordneten dritten Kammern 13 und die Lagen bzw. Matten 19, 21 der TPX- bzw. PES-Fasern sind in diesem Beispiel abwechselnd unmittelbar auf- bzw. übereinander geschichtet. Dies ergibt eine kompakte Fasermatte. Die Matten bzw. Fasermatten 19, 21 werden bevorzugt direkt aufeinander gelegt, sodass sie in Kontakt miteinander sind. In Figur 1 sind die Lagen bzw. Matten 19, 21 weit auseinander in einer Explosions- Darstellung gezeigt, um eine deutlichere Erklärung der Erfindung zu ermöglichen.

Es zeigt sich aus obigen Ausführungen, dass eine Verwendung der erfinderischen Vorrichtung für eine Vielzahl von Anwendungen möglich ist. Im Folgenden erfolgt eine detailliertere Beschreibung der Erfindung anhand einer bevorzugten Ausführungsform.

Gemäß Figur 2 ist die Vorrichtung 1 als eine künstliche Lunge ausgebildet, die in der Figur 2 schematisch dargestellt ist. Das Gehäuse 2 der Vorrichtung 1 ist dabei im Wesentlichen quaderförmig mit rundlich verlaufenden Seitenkanten ausgebildet. An der Einlassoberfläche 5 des Gehäuses 2 ist in einem Eckbereich der Einlassoberfläche 5 ein Einlassabschnitt 31 in Form eines Konnektors ausgebildet. An einer dem Gehäuse 2 abgewandten Seite des Einlassabschnitts 31 ist eine Zuleitung 33 ausgebildet. Auf ähnliche Weise ist auf der Auslassoberfläche 6 des Gehäuses 2 ein Auslassabschnitt 41 mit einer Ableitung 43 vorgesehen. Aufgrund der perspektivischen Darstellung der Figur 2 ist die Auslassoberfläche 6 in der Figur jedoch nicht zu erkennen.

Des Weiteren ist auf oder benachbart zu der Einlassoberfläche 5 zudem ein weiterer Anschluss 35 vorgesehen. Der Anschluss 35 zweigt insbesondere von einer Prismenfläche 4 des Gehäuses 2 ab. Der Anschluss 35 kann verschiedene Funktionen aufweisen. Beispielsweise kann der Anschluss 35 zur Gasversorgung des Gasaustauschmittels, also im Wesentlichen der zweiten Kammer 7 bzw. der dritten Kammer 13 entsprechend der in Figur 1 gezeigten Ausführungsform des Gasaustauschmittels, dienen. Zudem kann ein Fluidabtransport mittels des Anschlusses 35 erfolgen, insbesondere in einem Fluidkreislaufsystem mit mehreren Anschlüssen 35, wie in Figur 2 dargestellt.

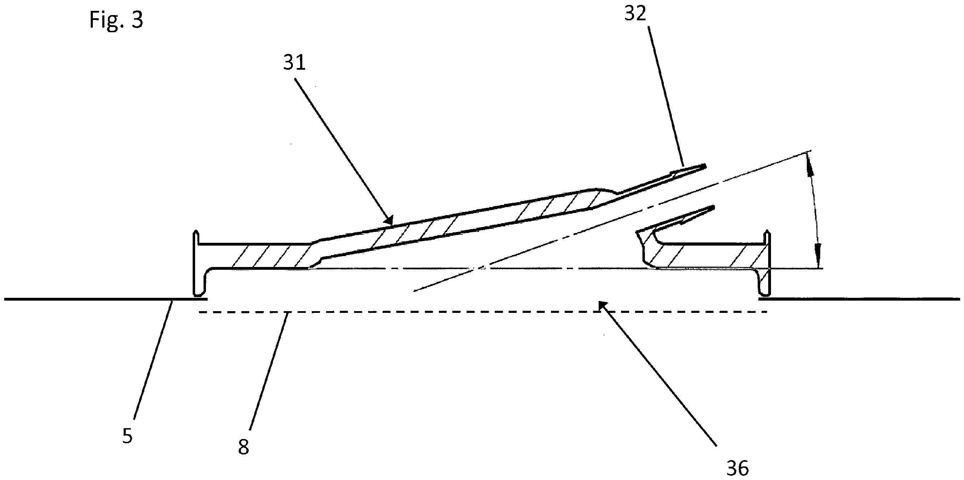

Figur 3 zeigt einen Einlassabschnitt 31 für eine erfindungsgemäße Vorrichtung 1. Der Einlassabschnitt 31 ist auf der Einlassoberfläche 5 des Gehäuses 2 ausgebildet. An einer der Einlassoberfläche 5 abgewandten Seite des Einlassabschnitts 31 ist ein Anschlussabschnitt 32 vorgesehen. Der Anschlussabschnitt 32 dient der Verbindung des Einlassabschnitts 31 mit einer Fluidzufuhr, also beispielsweise einem Schlauch als Zuleitung 33. In der hier dargestellten Ausführungsform dient der Einlassabschnitt dabei insbesondere der Zuleitung einer biologischen Flüssigkeit, wie beispielsweise Blut. In der Einlassoberfläche 5 ist eine Öffnung 36 vorgesehen, die den Einlass der Flüssigkeit in die erste Kammer 3 - nicht dargestellt in Figur 3 - bildet.

Wie aus Figur 3 ersichtlich, ist der Einlassabschnitt in einem spitzen Winkel relativ zu der Einlassoberfläche 5 ausgebildet und angeordnet. In der gezeigten Ausführungsform gemäß Figur 3 ist dieser Einlasswinkel ca. 15°. Der Einlasswinkel kann jedoch variiert werden, wobei der Einlasswinkel vorzugsweise kleiner als 45°, weiter vorzugsweise kleiner als 25°, und insbesondere zwischen 15° - 20° beträgt. Aufgrund der Parallelität der Oberfläche 8 des im Gehäuse 2 angeordneten Faserbündels 18 zur Einlassoberfläche 5 bzw. zu der durch diese definierten Ebene, verläuft der Einlassabschnitt 31 in einem spitzen Anströmwinkel α relativ zu der Oberfläche 8 des Faserbündels 18. Dieser Anströmwinkel α ist vorzugsweise kleiner als 45°, weiter vorzugsweise kleiner als 25°, und hat insbesondere einen Wert von 5° bis 20°. Im vorliegenden Ausführungsbeispiel hat der Anströmwinkel α einen Wert von 15°.

Auf diese Weise wird eine tangentiale Anströmung mit der biologischen Flüssigkeit ermöglicht. Zudem kann auf diese Weise eine tangentiale Zu- und Ableitungsführung ermöglicht werden. Dies kann wiederum einen Einbau in einem kompakten Format beispielsweise in einer tragbaren Vorrichtung erlauben.

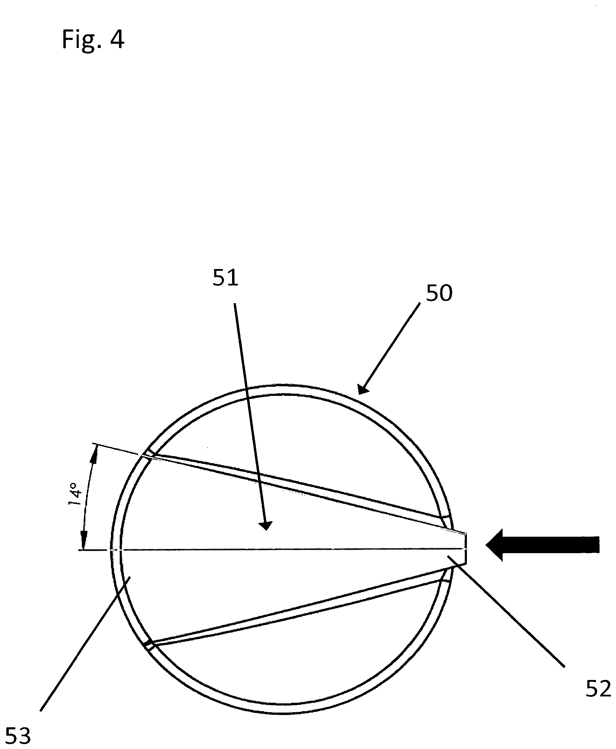

Figur 4 zeigt einen Deckel 50 der erfindungsgemäßen Vorrichtung. Der Deckel 50 ist dabei insbesondere derart ausgebildet, dass er unterhalb der in Figur 3 gezeigten Öffnung 36 angeordnet werden kann, so dass die durch den Einlassabschnitt 31 eingeleitete Flüssigkeit auf den Deckel 50 geleitet wird. Der Deckel 50 weist dabei einen kanalartigen Abschnitt 51 auf. "Kanalartig" ist dabei so zu verstehen, dass der kanalartige Abschnitt 51 eine Vertiefung in dem Deckel 50 darstellt, die eine Flussrichtung der auf den Deckel 50 geleiteten Flüssigkeit vorgibt. Der Pfeil in Figur 4 kennzeichnet dabei eine Flussrichtung der eingeleiteten Flüssigkeit entlang des Deckels 50.

Wie zudem aus Figur 4 entnehmbar ist, weist der kanalartige Abschnitt 51 ein erstes Ende 52 und ein zweites Ende 53 auf. Der kanalartige Abschnitt 51 ist derart ausgebildet, dass er sich in der Flussrichtung von dessen ersten Ende 52 zu dessen zweiten Ende 53 aufweitet. Dies führt zu einer Verteilung der eingeleiteten Flüssigkeit über eine größere Fläche und erlaubt somit eine homogenere Anströmung der dem Deckel 50 nachgeschalteten Komponenten, also im Wesentlichen der ersten Kammer 3 mit dem Gasaustauschmittel.

Der Deckel 50 bzw. der kanalartige Abschnitt 51 des Deckels 50 ist dabei vorzugsweise derart ausgebildet, dass sich der kanalartige Abschnitt 51 in einem Winkel von zwischen 10°-20° aufweitet. In der in Figur 4 gezeigten Ausführungsform ist der Aufspannwinkel, also der Winkel, um den sich der kanalartige Abschnitt 51 nach dieser Definition aufweitet, 14°. Der Winkel, den die einzelnen Schenkel des kanalartigen Abschnitts 51 einschließen ist der doppelte Aufspannwinkel, also 28° in der gezeigten Ausführungsform.

Während in den in Figur 3 und 4 gezeigten Ausführungsformen der Einlassabschnitt 31 und der Deckel 50 als einzelne Komponenten ausgebildet sind, ist es denkbar, dass der Einlassabschnitt 31 auch einstückig mit dem Deckel 50 ausgebildet ist. In diesem Falle könnte der Deckel 50 beispielsweise in der Öffnung 36 der Einlassoberfläche 5 so angeordnet sein, dass die durch den Einlassabschnitt 31 eingeleitete Flüssigkeit direkt in die Kammer 3 eingeleitet wird. Dabei kann der Deckel auch eine schräge Ebene aufweisen, die, beispielsweise der Neigung des Einlassabschnitts folgend, von der Einlassoberfläche 5 durch die Öffnung 36 in die erste Kammer führt.

Zudem ist in der Ausführungsform gemäß Figur 4 der Deckel aufgrund des sich aufweitenden Kanals auch gleichzeitig ein Druckmittler-Bauteil, da die anströmende Flüssigkeit auf eine größere Fläche verteilt und somit der Strömungsdruck verringert wird. Wiederum gleichzeitig stellt der Deckel gemäß der Ausführung nach Figur 4 auch ein Verteilermittel für die anströmende Flüssigkeit dar.

In alternativen Ausführungsformen kann der Deckel 50 auch separat von einem Druckmittler oder von einem Verteilermittel ausgebildet sein, beispielsweise lediglich als eine Abdeckung der Einlassoberfläche 5 an der Außenseite der Einlassoberfläche 5 oder an der Innenseite der Einlassoberfläche 5. In diesem Falle könnten ein separater Druckmittler und/oder ein separates Verteilermittel in der Vorrichtung vorgesehen sein.

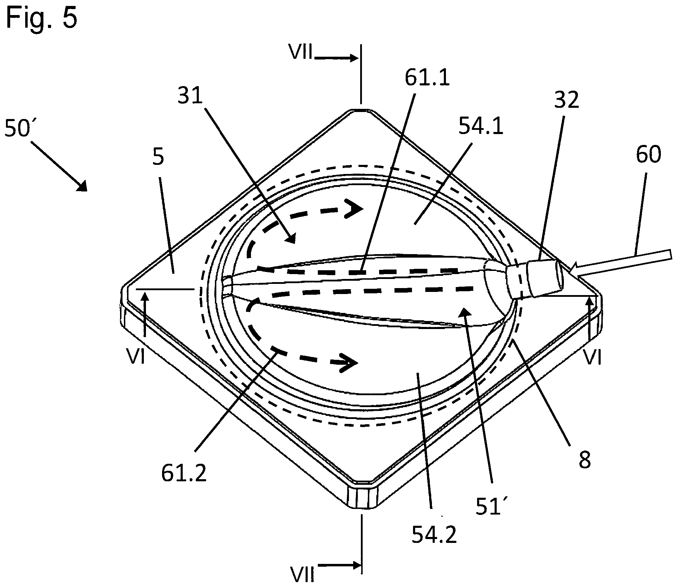

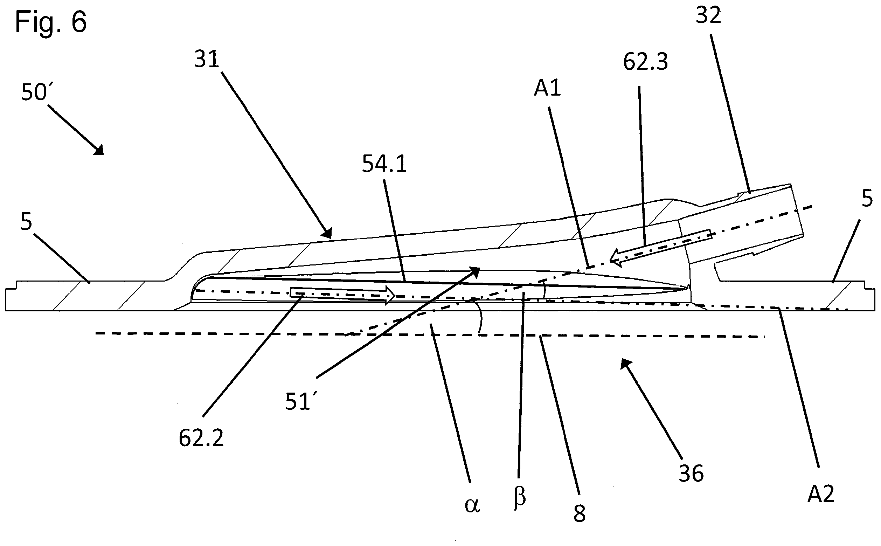

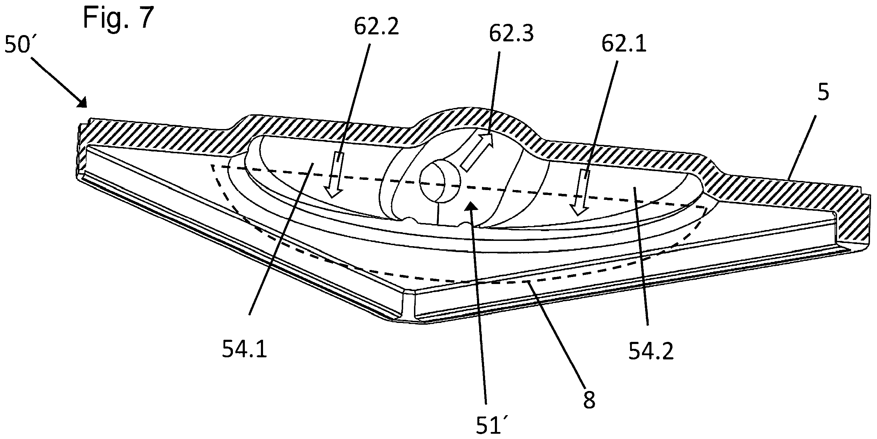

In den Figuren 5 bis 7 ist ein alternativer Deckel 50' der erfindungsgemäßen Vorrichtung dargestellt. Der Deckel 50' ist entweder so ausgebildet, dass er unterhalb der in Figur 3 gezeigten Öffnung 36 angeordnet werden kann, so dass die durch den Einlassabschnitt 31 eingeleitete Flüssigkeit auf den Deckel 50' geleitet wird, oder er ist so ausgebildet dass er die gesamte Oberfläche/Einlassoberfläche 5 des prismatischen Gehäuses 2 formt. Der Deckel 50' weist dabei wiederum einen kanalartigen Abschnitt 51' auf. "Kanalartig" ist dabei so zu verstehen, dass der kanalartige Abschnitt 51' eine Aufwölbung in dem Deckel 50' darstellt, die eine Flussrichtung der in den Deckel 50' eingeleiteten Flüssigkeit vorgibt. Der Pfeil 60 in Figur 5 kennzeichnet dabei eine Einströmrichtung einer in den Deckel 50' eingeleiteten Flüssigkeit und die Pfeile 61.1 und 61.2 (in Fig. 5) und die Pfeile 62.1 bis 62.3 (Figuren 6 und 7) den weiteren Strömungsverlauf der einströmenden Flüssigkeit zwischen dem Deckel 50'und dem darunter, innen im Gehäuse liegenden Faserbündel, welches in den Figuren 5 bis 7 durch die Lage seiner Oberfläche 8 angedeutet ist.

Bei dieser Ausführungsform ist der kanalartigen Abschnitt 51' des Einlassabschnitts 31' in einem spitzen Einlasswinkel relativ zu der Einlassoberfläche 5 angeordnet, wie insbesondere aus Figur 6 ersichtlich ist. In der gezeigten Ausführungsform gemäß Figur 6 ist dieser Einlasswinkel ca. 15°. Der Einlasswinkel kann jedoch variiert werden, wobei der Einlasswinkel vorzugsweise kleiner als 45°, weiter vorzugsweise kleiner als 25°, und insbesondere zwischen 15° - 20° beträgt.

Aufgrund der Parallelität der Oberfläche 8 des im Gehäuse 2 angeordneten Faserbündels 18 zur Einlassoberfläche 5 bzw. zu der durch diese definierten Ebene, verläuft der kanalartige Abschnitt 50' des Einlassabschnitts 31' in einem spitzen Anströmwinkel α relativ zu der Oberfläche 8 des Faserbündels 18. Dieser Anströmwinkel α ist vorzugsweise kleiner als 45°, weiter vorzugsweise kleiner als 25°, und hat insbesondere einen Wert von 5° bis 20°. Im vorliegenden Ausführungsbeispiel hat der Anströmwinkel α einen Wert von 15°.

Zu beiden Seiten des kanalartigen Abschnitts 50' befinden sich jeweils Strömungs-Leitflächen 54.1 und 54.2, die ein gegenläufiges Gefälle (Pfeil 62.2 in Fig. 6) zu dem Gefälle (Pfeil 62.3 in Fig. 6) des kanalartigen Abschnitts 50' aufweisen, so dass sich eine durch den kanalartigen Abschnitt 50' definierte erste Achse A1 und eine durch die Strömungs-Leitflächen 54.1 und 54.2 definierte zweite Achse A2 in einem spitzen Winkel β von ca. 5° bis 20° schneiden. Im vorliegenden Ausführungsbeispiel weist der spitze Winkel β einen Wert von 16,35° auf. Das Gefälle der beiden Strömungs-Leitflächen 54.1 und 54.2 (Pfeil 62.2 in Fig. 6) beträgt vorzugsweise 1° bis 5°. Im vorliegenden Ausführungsbeispiel beträgt das Gefälle (welches dem Winkel der Strömungs-Leitflächen 54.1 und 54.2 zur Oberfläche 8 des Faserbündels 18 entspricht) 1,35°. Durch die beiden Strömungs-Leitflächen 54.1 und 54.2 wird eine wirbelfreie Aufteilung des einströmenden Fluidstromes erreicht. In dem Fall, dass das eingeleitete Fluid Blut ist, wird eine Blutgerinnung (Verklumpung) und eine Blutschädigung (Hämolyse) effektiv verhindert. Es wird eine sehr gute dauerhafte Gasaustauscheffektivität bezogen auf die Gasaustauschfläche erreicht.

Wie der Fachmann im Übrigen verstehen wird, können weitere Anschlüsse auf dieselbe oder auf ähnliche Weise an der Oberfläche der Vorrichtung vorgesehen sein. Der Aufbau der erfindungsgemäßen Vorrichtung erlaubt somit eine Mehrzahl von möglichen gleichzeitigen Behandlungen einer Flüssigkeit, insbesondere einer biologischen Flüssigkeit.

Gemäß einer speziellen Ausführung der vorliegenden Erfindung soll mit der erfindungsgemäßen Vorrichtung lediglich eine Decarboxylierung ohne gleichzeitige Oxygenierung vorgenommen werden. Da es ein grundsätzliches Bestreben ist, extrakorporale Volumenströme so gering wie möglich zu halten, beispielsweise bei der Entnahme und Zurückleitung von Blut in einen Körper, liegen die Volumenströme für derartige CO2 Entfernungs-Systeme typischerweise im Bereich unter 2l/min, bevorzugt bei ca. 0,3-1 l/min. Um bei derart geringen Volumenströmen eine suffiziente Durchströmung und einen effektiven Gasaustausch zu gewährleisten ist die Dimensionierung des Faserbündels bzw. der Kavität entscheidend. Bei der Optimierung der Dimensionierung ist ein Kompromiss zu finden zwischen den konkurrierenden Anforderungen wie einer großer Gasaustauschoberfläche zum effektiven Gasaustausch, einer kompakten Bauweise zur besseren Mobilität und Tragbarkeit, einer homogenen Strömungsverteilung und somit einem geringen Druckverlust und geringen Scherkräften, sowie von Hämokompatibilität zur Erhöhung der Einsatzdauer des Gerätes.

Aus diesen Überlegungen und entsprechenden Berechnungen wurde gemäß einer Ausführungsform der vorliegenden Erfindung für die Kavität des Gasaustauschers, also die erste Kammer, ein Durchmesser von 70mm und eine Stapeldicke des Gasaustauschmittels, hier des Faserbündels, von 25mm gewählt. Daraus ergibt sich in dieser besonderen Ausführungsform eine Gasaustauschoberfläche von ca. 0,6m². Diese Dimensionen stellen ein mögliches, akzeptables Verhältnis hinsichtlich der auftretenden Scherkräfte und der Druckverluste im Gasaustauscher dar. Ferner führen diese Abmessungen zu einer ausreichenden CO₂ Reduktion bei niedrigen Volumenströmen.

Es versteht sich jedoch, dass die vorliegende Erfindung sich nicht lediglich auf eine Decarboxylierung beschränkt.

### Bezugszeichenliste

- 1: Gasaustauschvorrichtung
- 2: Gehäuse
- 3: erste Kammer
- 4: Prismenflächen (des Gehäuses)
- 5: Einlassoberfläche/Oberfläche des Gehäuses
- 6: Auslassoberfläche
- 7: zweite Kammer
- 8: Oberfläche des Faserbündels
- 9: Membran
- 10: Pfeil
- 11: Einlass
- 12: Auslass
- 13: dritte Kammer
- 14: Wand
- 15: Membran
- 18: Faserbündel
- 19: Fasermatte
- 21: Fasermatte
- 31: Einlassabschnitt
- 32: Anschlussabschnitt
- 33: Zuleitung
- 35: Anschluss
- 36: Öffnung
- 41: Auslassabschnitt
- 43: Ableitung
- 50: Deckel
- 50': Deckel
- 51: kanalartiger Abschnitt
- 51': kanalartiger Abschnitt
- 52: erstes Ende
- 53: zweites Ende
- 54.1: Strömungs-Leitfläche
- 54.2: Strömungs-Leitfläche
- 60: Pfeil
- 61.1: Pfeil
- 61.2: Pfeil
- 62.1: Pfeil (Strömungsrichtung)
- 62.2: Pfeil (Strömungsrichtung/Gefälle)
- 62.3: Pfeil (Strömungsrichtung/Gefälle)
- A1: erste Achse
- A2: zweite Achse
- α: Anströmwinkel
- β: spitzer Winkel

## Patentansprüche

1. Vorrichtung (1) zur Behandlung einer biologischen Flüssigkeit, umfassend ein Gehäuse (2) mit einer ersten, eine Kavität bildenden Kammer (3), die zur Aufnahme der zu behandelnden Flüssigkeit ausgebildet ist, und wenigstens ein Gasaustauschmittel (7, 13, 19, 21), das als ein durch eine Anordnung mehrerer übereinander geschichteter Fasermatten (19, 20) aus einer Mehrzahl von Hohlfasern gebildetes Faserbündel (18) ausgebildet ist, das zumindest teilweise in der ersten Kammer (3) angeordnet ist, wobei in einer Einlassoberfläche (5) des Gehäuses (2) ein Einlassabschnitt (31) zum Einlass der zu behandelnden Flüssigkeit in die erste Kammer (3) ausgebildet ist, wobei der Einlassabschnitt (31) in einem spitzen Winkel relativ zu der Einlassoberfläche (5) des Gehäuses (2) ausgebildet ist,
**dadurch gekennzeichnet, dass** der Einlassabschnitt (31) in einem spitzen Anströmwinkel (α) relativ zu einer Oberfläche (8) des Faserbündels (18) verläuft, die durch eine erste Fasermatte (19, 20) definiert ist und die sich im Wesentlichen parallel zu einer durch die Einlassoberfläche (5) definierten Ebene erstreckt.

2. Vorrichtung (1) gemäß Anspruch 1, **dadurch gekennzeichnet, dass** das Gasaustauschmittel wenigstens eine zweite Kammer (7) aufweist, wobei die erste Kammer (3) und die zweite Kammer (7) durch mindestens eine semi-permeable Membran (9) voneinander getrennt ausgebildet sind, und wobei zumindest eine vorbestimmte Molekülart zwischen der ersten Kammer (3) und der zweiten Kammer (7) zur Behandlung der biologischen Flüssigkeit durch die Membran (9) transferierbar ist.

3. Vorrichtung (1) gemäß Anspruch 1 oder 2, **dadurch gekennzeichnet, dass** das Gehäuse (2) des Weiteren einen Auslassabschnitt (41) an einer Auslassoberfläche (6) des Gehäuses (2) zum Auslassen der biologischen Flüssigkeit aus der Vorrichtung (1) aufweist, wobei der Auslassabschnitt (41) in einem spitzen Winkel relativ zu der Auslassoberfläche (6) des Gehäuses (2) angeordnet ist.

4. Vorrichtung (1) gemäß Anspruch 3, **dadurch gekennzeichnet, dass** der Winkel des Auslassabschnitts (41) relativ zu der Auslassoberfläche (6) des Gehäuses (2) kleiner als 45°, kleiner als 25°, oder zwischen 15° - 20° beträgt.

5. Vorrichtung (1) gemäß einem der vorhergehenden Ansprüche, **dadurch gekennzeichnet, dass** der Winkel des Einlassabschnitts (31) relativ zu der Einlassoberfläche (5) des Gehäuses (2) kleiner als 45°, kleiner als 25°, oder zwischen 15° - 20° beträgt.

6. Vorrichtung (1) gemäß einem der vorhergehenden Ansprüche, **dadurch gekennzeichnet, dass** der Einlassabschnitt (31) derart an der Einlassoberfläche (5) des Gehäuses (2) ausgebildet ist, dass eine durch den Einlassabschnitt (31) geleitete biologische Flüssigkeit in einem zentralen Bereich des Gasaustauschmittels in die erste Kammer (3) einleitbar ist.

7. Vorrichtung (1) gemäß einem der Ansprüche 4 bis 6, **dadurch gekennzeichnet, dass** die erste Kammer (3) und/oder das Gasaustauschmittel eine im Wesentlichen zylindrische Form aufweist.

8. Vorrichtung (1) gemäß einem der vorhergehenden Ansprüche, **dadurch gekennzeichnet, dass** das Gehäuse (2) eine prismatische Form aufweist, bei der die Seitenflächen (4) des Gehäuses (2) als Prismenflächen ausgebildet sind, wobei der Habitus des Gehäuses (2) gedrungen ist und wobei der Einlassabschnitt (31) an einer als Einlassoberfläche (5) ausgebildeten Stirnfläche des prismatischen Gehäuses (2) angeordnet ist.

9. Vorrichtung (1) gemäß einem der vorhergehenden Ansprüche, **dadurch gekennzeichnet, dass** in der ersten Kammer (3) an einer dem Einlassabschnitt (31) zugewandten Seite ein Druckmittler zum Ausgleichen von Druckunterschieden in einem Strömungsquerschnitt der biologischen Flüssigkeit ausgebildet ist.

10. Vorrichtung (1) gemäß Anspruch 9, **dadurch gekennzeichnet, dass** der Druckmittler eine im Wesentlichen schräge Ebene aufweist, entlang derer die biologische Flüssigkeit beim Einfließen in die Vorrichtung (1) geleitet ist.

11. Vorrichtung (1) gemäß einem der vorherigen Ansprüche, **dadurch gekennzeichnet, dass** die Einlassoberfläche (5) und/oder die Auslassoberfläche (6) der Vorrichtung (1) einen Deckel (50) darstellt oder einen Deckel (50) aufweist.

12. Vorrichtung (1) gemäß einem der vorhergehenden Ansprüche, **dadurch gekennzeichnet, dass** in der Vorrichtung (1) ein Verteilermittel ausgebildet ist, das ausgebildet ist, die biologische Flüssigkeit in einer zur Strömungsrichtung im Wesentlichen lateralen Richtung zu verteilen.

13. Vorrichtung (1) gemäß Anspruch 12, **dadurch gekennzeichnet, dass** das Verteilermittel ausgehend von einer dem Einlassabschnitt (31) zugewandten Seite einen kanalartigen Abschnitt (51) aufweist, der sich in der Strömungsrichtung der biologischen Flüssigkeit in lateraler Richtung öffnet.

14. Vorrichtung (1) gemäß Anspruch 12 oder 13, **dadurch gekennzeichnet, dass** das Verteilermittel einstückig mit dem Druckmittler und/oder dem Einlassabschnitt (31) und/oder dem Deckel (50) und/oder der Einlassoberfläche (5) des Gehäuses (2) ausgebildet ist.

15. Vorrichtung (1) gemäß einem der vorhergehenden Ansprüche, **dadurch gekennzeichnet, dass** in der ersten Kammer (3) eine dritte Kammer (13) ausgebildet ist, die durch mindestens eine flüssigkeitsdurchlässige Membran (15) von der ersten Kammer (3) getrennt ist und einem Entzug einer oder mehrerer Komponenten der biologischen Flüssigkeit dient.

16. Vorrichtung (1) nach Anspruch 13, **dadurch gekennzeichnet, dass** zu beiden Seiten des kanalartigen Abschnitts (51') Strömungs-Leitflächen (54.1, 54.2) angeordnet sind, die ein gegenläufiges Gefälle (62.2) zu dem Gefälle (62.3) des kanalartigen Abschnitts (51') aufweisen, so dass sich eine durch den kanalartigen Abschnitt (51') definierte erste Achse (A1) und eine durch die Strömungs-Leitflächen (54.1) und (54.2) definierte zweite Achse (A2) in einem spitzen Winkel (β) schneiden.

17. Vorrichtung (1) nach Anspruch 16, **dadurch gekennzeichnet, dass** der spitze Winkel (β) 5° bis 20° beträgt.

## Claims

1. A device (1) for treating a biological liquid, comprising a housing (2) with a first chamber (3) defining a cavity, which is adapted to receive the liquid to be treated, and at least one gas exchanger (7, 13, 19, 21) formed as a fiber bundle (18) of a plurality of hollow fibers by an arrangement of a plurality of layered fiber mats (19, 20), which is at least partially disposed in the first chamber (3), wherein an inlet portion (31) for inlet of the liquid to be treated into the first chamber (3) is formed in an inlet surface (5) of the housing (2), wherein the inlet portion (31) is formed at an acute angle relative to the inlet surface (5) of the housing (2),
**characterized in that** the inlet portion (31) extends at an acute inflow angle (α) relative to a surface (8) of the fiber bundle (18), which is defined by a first fiber mat (19, 20) and which extends essentially parallel to a plane defined by the inlet surface (5).

2. Device (1) according to claim 1, **characterized in that** the gas exchanger comprises at least a second chamber (7), wherein the first chamber (3) and the second chamber (7) are separated from each other by at least one semi-permeable membrane (9), and wherein at least one predetermined molecule type can be transferred between the first chamber (3) and the second chamber (7) through the membrane (9) for treatment of the biological liquid.

3. Device (1) according to claim 1 or 2, **characterized in that** the housing (2) further comprises an outlet portion (41) at an outlet surface (6) of the housing (2) for the outlet of the biological liquid from the device (1), wherein the outlet portion (41) is arranged at an acute angle relative to the outlet surface (6) of the housing (2).

4. Device (1) according to claim 3, **characterized in that** the angle of the outlet portion (41) relative to the outlet surface (6) of the housing (2) is less than 45°, less than 25°, or between 15° - 20°.

5. Device (1) according to any one of the preceding claims, **characterized in that** the angle of the inlet portion (31) relative to the inlet surface (5) of the housing (2) is less than 45°, less than 25°, or between 15° - 20°.

6. Device (1) according to any one of the preceding claims, **characterized in that** the inlet portion (31) is formed on the inlet surface (5) of the housing (2) such that a biological liquid guided through the inlet portion (31) can be guided into the first chamber (3) in a central region of the gas exchanger.

7. Device (1) according to any one of claims 4 to 6, **characterized in that** the first chamber (3) and/or the gas exchanger has a substantially cylindrical shape.

8. Device (1) according to any one of the preceding claims, **characterized in that** the housing (2) has a prismatic shape, in which the side surfaces (4) of the housing (2) are formed as prism surfaces, wherein the shape of the housing (2) is compact and wherein the inlet portion (31) is arranged on an end face of the prismatic housing (2) formed as an inlet surface (5).

9. Device (1) according to any one of the preceding claims, **characterized in that** a diaphragm seal adapted to balance a pressure differences in a flow cross-section of the biological liquid is disposed on a side of the first chamber (3) facing the inlet portion (31).

10. Device (1) according to claim 9, **characterized in that** the diaphragm seal defines an essentially oblique plane, along which the biological liquid is guided when flowing into the device (1).

11. Device (1) according to any one of the preceding claims, **characterized in that** the inlet surface (5) and/or the outlet surface (6) of the device (1) is or comprises a cover (50).

12. Device (1) according to any one of the preceding claims, **characterized in that** a distributor is arranged in the device (1), which is adapted to distribute the biological liquid in a direction essentially lateral to the flow direction.

13. Device (1) according to claim 12, **characterized in that** the distributor has a channel-like portion (51), extending from a side facing the inlet portion (31), the channel-like portion (51) opening in the lateral direction to the flow direction of the biological liquid.

14. Device (1) according to claim 12 or 13, **characterized in that** the distributor is formed integrally with the diaphragm seal and/or the inlet portion (31) and/or the cover (50) and/or the inlet surface (5) of the housing (2).

15. Device (1) according to any one of the preceding claims, **characterized in that** a third chamber (13) is disposed in the first chamber (3), which is separated from the first chamber (3) by at least one liquid-permeable membrane (15) and which is adapted to extract one or more components of the biological liquid.

16. Device (1) according to claim 13, **characterized in that** flow guiding surfaces (54.1, 54.2) are arranged on both sides of the channel-like portion (51'), the flow guiding surfaces (54.1, 54.2) having a slope (62.2) opposite to a slope (62. 3) of the channel-like portion (51'), so that a first axis (A1) defined by the channel-like portion (51') and a second axis (A2) defined by the flow guiding surfaces (54.1) and (54.2) intersect at an acute angle (β).

17. Device (1) according to claim 16, **characterized in that** the acute angle (β) is 5° to 20°.

## Revendications

1. Dispositif (1) destiné au traitement d'un liquide biologique, qui comprend un boîtier (2) comportant une première chambre (3) qui forme une cavité, qui est réalisée à des fins de réception du liquide qui doit être soumis à un traitement, et au moins un moyen d'échange de gaz (7, 13, 19, 21) qui est réalisé sous la forme d'un faisceau de fibres (18) formé par l'intermédiaire d'un agencement de plusieurs mats de fibres superposés les uns aux autres (19, 20) réalisés à partir d'une multitude de fibres creuses, le faisceau en question étant disposé au moins en partie dans la première chambre (3) ; dans lequel, dans une surface d'entrée (5) du boîtier (2), est réalisé un tronçon d'entrée (31) pour l'amenée, dans la première chambre (3), du liquide qui doit être soumis à un traitement ; dans lequel le tronçon d'entrée (31) est réalisé en formant un angle aigu par rapport à la surface d'entrée (5) du boîtier (2), **caractérisé en ce que** le tronçon d'entrée (31) s'étend en formant un angle d'incidence aigu (α) par rapport à une surface (8) du faisceau de fibres (18), qui est définie par un premier mat de fibres (19, 20) et qui s'étend de manière essentielle parallèlement à un plan qui est défini par la surface d'entrée (5).

2. Dispositif (1) selon la revendication 1, **caractérisé en ce que** le moyen d'échange de gaz présente au moins une deuxième chambre (7) ; dans lequel la première chambre (3) et la deuxième chambre (7) sont réalisées de manière à être séparées l'une de l'autre par l'intermédiaire d'au moins une membrane semiperméable (9) ; et dans lequel on peut transférer au moins un type de molécule qui a été déterminé au préalable entre la première chambre (3) et la deuxième chambre (7) à des fins de traitement du liquide biologique, à travers la membrane (9).

3. Dispositif (1) selon la revendication 1 ou 2, **caractérisé en ce que** le boîtier (2) présente en outre un tronçon de sortie (41) contre une surface de sortie (6) du boîtier (2), à des fins d'évacuation du liquide biologique à l'extérieur du dispositif (1) ; dans lequel le tronçon de sortie (41) est disposé en formant un angle aigu par rapport à la surface de sortie (6) du boîtier (2).

4. Dispositif (1) selon la revendication 3, **caractérisé en ce que** l'angle formé par le tronçon de sortie (41) par rapport à la surface de sortie (6) du boîtier (2) est inférieur à 45°, inférieur à 25° ou se situe entre 15° et 20°.

5. Dispositif (1) selon l'une quelconque des revendications précédentes, **caractérisé en ce que** l'angle formé par le tronçon d'entrée (31) par rapport à la surface d'entrée (5) du boîtier (2) est inférieur à 45°, inférieur à 25° ou se situe entre 15° et 20°.

6. Dispositif (1) selon l'une quelconque des revendications précédentes, **caractérisé en ce que** le tronçon d'entrée (31) est réalisé contre la surface d'entrée (5) du boîtier (2) d'une manière telle que l'on peut introduire de manière guidée, dans la première chambre (3), un liquide biologique qui est guidé à travers le tronçon d'entrée (31) dans une zone centrale du moyen d'échange de gaz.

7. Dispositif (1) selon l'une quelconque des revendications 4 à 6, **caractérisé en ce que** la première chambre (3) et/ou le moyen d'échange de gaz présente(nt) une forme essentiellement cylindrique.

8. Dispositif (1) selon l'une quelconque des revendications précédentes, **caractérisé en ce que** le boîtier (2) présente une forme prismatique dans laquelle les faces latérales (4) du boîtier (2) sont réalisées sous la forme de faces de prisme ; dans lequel la conformation du boîtier (2) est trapue ; et dans lequel le tronçon d'entrée (31) est disposé contre une surface avant du boîtier prismatique (2), qui est réalisée sous la forme d'une surface d'entrée (5).

9. Dispositif (1) selon l'une quelconque des revendications précédentes, **caractérisé en ce que**, dans la première chambre (3), contre un côté qui est orienté dans la direction du tronçon d'entrée (31), est réalisé un transmetteur de pression destiné à l'équilibrage des différences de pression qui règnent dans une section transversale d'écoulement du liquide biologique.

10. Dispositif (1) selon la revendication 9, **caractérisé en ce que** le transmetteur de pression présente un plan essentiellement incliné le long duquel est guidé le liquide biologique lors de son entrée sous la forme d'un écoulement dans le dispositif (1).

11. Dispositif (1) selon l'une quelconque des revendications précédentes, **caractérisé en ce que** la surface d'entrée (5) et/ou la surface de sortie (6) du dispositif (1) représente(nt) un couvercle (50) ou présente(nt) un couvercle (50).

12. Dispositif (1) selon l'une quelconque des revendications précédentes, **caractérisé en ce que**, dans le dispositif (1), est réalisé un moyen de répartition qui est réalisé pour répartir le liquide biologique dans une direction essentiellement latérale par rapport à la direction d'écoulement.

13. Dispositif (1) selon la revendication 12, **caractérisé en ce que** le moyen de répartition présente, à partir d'un côté orienté dans la direction du tronçon d'entrée (31), un tronçon en forme de canal (51) qui s'ouvre en direction latérale dans la direction d'écoulement du liquide biologique.

14. Dispositif (1) selon la revendication 12 ou 13, **caractérisé en ce que** le moyen de répartition est réalisé en une seule pièce avec le transmetteur de pression et/ou avec le tronçon d'entrée (31) et/ou avec le couvercle (50) et/ou avec la surface d'entrée (5) du boîtier (2).

15. Dispositif (1) selon l'une quelconque des revendications précédentes, **caractérisé en ce que**, dans la première chambre (3), est réalisée une troisième chambre (13) qui est séparée de la première chambre (3) par au moins une membrane perméable aux liquides (15) et qui sert à une extraction d'un ou de plusieurs composants du liquide biologique.

16. Dispositif (1) selon la revendication 13, **caractérisé en ce que**, des deux côtés du tronçon en forme de canal (51'), sont disposées des surfaces de guidage de l'écoulement (54.1, 54.2) qui présentent une déclivité (62.2) dans le sens contraire de celle de la déclivité (62.3) du tronçon en forme de canal (51'), d'une manière telle qu'un premier axe (A1) qui est défini par le tronçon en forme de canal (51') et un deuxième axe (A2) qui est défini par les surfaces de guidage de l'écoulement (54.1) et (54.2) se coupent en formant un angle aigu (β).

17. Dispositif (1) selon la revendication 16, **caractérisé en ce que** l'angle aigu (β) s'élève de 5° à 20°.
